# EUROPEAN PATENT SPECIFICATION

(11) **EP 0 578 611 B1**
(45) Date of publication and mention of the grant of the patent: **26.11.2003**
(21) Application number: 93810455.1
(22) Date of filing: 24.06.1993
(51) Int. Cl.: C12N 15/82, C12N 15/29, C12N 1/21, C12N 5/10, A01H 5/00

(54) **Anther-specific cDNA sequences, genomic DNA sequences and recombinant DNA sequences**
Anthere-spezifische cDNA-Sequenzen, genomische DNA-sequenzen und rekombinante DNA-sequenzen
Séquences cDNA spécifiques pour des anthères, séquences d'ADN génomiques et séquences d'ADN récombinantes

(30) Priority: 02.07.1992 US 908242
(43) Date of publication of application: 12.01.1994
(73) Proprietor: Syngenta Participations AG, 4058 Basel (CH)
(72) Inventor: Tuttle, Annmarie B., Garner, NC 27529 (US); Crossland, Lyle D., Chapel Hill, NC 27514 (US)

(56) References cited:
- EP-A- 0 344 029
- THE PLANT CELL. vol. 2, no. 12, December 1990, ROCKVILLE, MD, USA. pages 1201 - 1224 KOLUTNOW, A.M., ET AL. 'Different temporal and spatial gene expression patterns occur during anther development'
- THE PLANT CELL. vol. 2, no. 12, December 1990, ROCKVILLE, MD, USA. pages 1201 - 1224 KOLUTNOW, A.M., ET AL. 'Different temporal and spatial gene expression patterns occur during anther development'
- NATURE vol. 347, 25 October 1990, LONDON GB pages 737 - 741 MARIANI, C., ET AL. 'Induction of male sterility in plants by a chimaeric ribonuclease gene'
- J. CELL. BIOCHEM. SUPPL., SYMPOSIUM ON EVOLUTION AND PLANT DEVELOPMENT, JAN.26 FEB.1, 1993. vol. 17B, 1993, page 24 TUTTLE, A., ET AL. 'Expression of tobacco anther-specific genes'

### SEQUENCE LISTING

(1) GENERAL INFORMATION:
   (i) APPLICANT:
      (A) NAME: CIBA-GEIGY AG
      (B) STREET: Klybeckstr. 141
      (C) CITY: Basel
      (E) COUNTRY: SCHWEIZ
      (F) POSTAL CODE (ZIP): 4002
      (G) TELEPHONE: +41 61 69 11 11
      (H) TELEFAX: + 41 61 696 79 76
      (I) TELEX: 962 991
   (ii) TITLE OF INVENTION: Anther-specific cDNA Sequences, Genomic DNA
      Sequences and Recombinant DNA Sequences
   (iii) NUMBER OF SEQUENCES: 21
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO:1:
   (i) SEQUENCE CHARACTERISTICS:
      (A) LENGTH: 1542 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant32
   (ix) FEATURE:
      (A) NAME/KEY: CDS
      (B) LOCATION: 66..1412
   (xi) SEQUENCE DESCRIPTION: SEQ ID NO:1:
   TCTGTCAAGA TAACAATAAA AGAATAAAAA GATTAACCAA AAACGATATA CATATTTAGG  60
   ACAGA ATG AAG GTT AGC TTG AAG CAC CAC TGG GTA GTG AAG CCA GCA     107
         Met Lys Val Ser Leu Lys His His Trp Val Val Lys Pro Ala
          1               5                   10
   GAG GCA ACA TGG AAT GGC ACT GTC TCC TTA TCG GAG TGT GAT CAA ACT   155
   Glu Ala Thr Trp Asn Gly Thr Val Ser Leu Ser Glu Cys Asp Gln Thr
    15                  20                  25                  30
   TTT GCT GTA ACT CAT GTA CCA ACC ATT TAT TAC TAC AGG TTT TGC CAT   203
   Phe Ala Val Thr His Val Pro Thr Ile Tyr Tyr Tyr Arg Phe Cys His
                    35                  40                  45
   GAT TGT CTT CCA TCA ACA GAC AAT ATC ATC AAA ACC CTC AGG ACC TCA   251
   Asp Cys Leu Pro Ser Thr Asp Asn Ile Ile Lys Thr Leu Arg Thr Ser
                50                  55                  60
   CTA AGC AAA GCA TTA GTA CAC TTC TAT CCA TTG TCT GGT CGT TTG CGA   299
   Leu Ser Lys Ala Leu Val His Phe Tyr Pro Leu Ser Gly Arg Leu Arg
           65                  70                  75
   TGG ATC GCT GGG TCC CGC CTC GAG CTC GAC TGT AAT GCC TCG GGA ATC   347
   Trp Ile Ala Gly Ser Arg Leu Glu Leu Asp Cys Asn Ala Ser Gly Ile
       80                  85                  90
   GTG CTC ATG GAA GCT GAA ACC GAA GCC AAA CTA GAT GAT CTT GGC GAT   395
   Val Leu Met Glu Ala Glu Thr Glu Ala Lys Leu Asp Asp Leu Gly Asp
    95                 100                 105                 110
   TTC TCG CCA TCC CCT GAC TTG AAC AGC TTG TTT CCC CGT GTA GAC TAC   443
   Phe Ser Pro Ser Pro Asp Leu Asn Ser Leu Phe Pro Arg Val Asp Tyr
                   115                 120                 125
   ACA ATC CCA ATT GAT GAA CTC CCT TTG TTG TTT GTT CAG CTT ACT AAG   491
   Thr Ile Pro Ile Asp Glu Leu Pro Leu Leu Phe Val Gln Leu Thr Lys
               130                 135                 140
   TTT CAG TGT GGT GGT ATT GCT CTG AGT TTT GCA ATA TCA CAT GCT GTA   539
   Phe Gln Cys Gly Gly Ile Ala Leu Ser Phe Ala Ile Ser His Ala Val
           145                 150                 155
   GTT GAT GGC CAA AGT GCT CTT TAC TTC CTC ACC GAA TGG GCT AGC CTT   587
   Val Asp Gly Gln Ser Ala Leu Tyr Phe Leu Thr Glu Trp Ala Ser Leu
       160                 165                 170
   GCT CGC GGA GAG CCA TTA GGG AAC GAA CCT TTT CAT GAT CGA AAA TTC   635
   Ala Arg Gly Glu Pro Leu Gly Asn Glu Pro Phe His Asp Arg Lys Phe
   175                 180                 185                 190
   CTC CGA GCA GGG GAA CCT CCA ATT GCA TAT CCA ACG TTT GAG CAT TTA   683
   Leu Arg Ala Gly Glu Pro Pro Ile Ala Tyr Pro Thr Phe Glu His Leu
                   195                 200                 205
   CAG TTT AAT CCA CCA CCA CTT TTG CTT GGA CAG TCC AGC AGT GAA GAG   731
   Gln Phe Asn Pro Pro Pro Leu Leu Leu Gly Gln Ser Ser Ser Glu Glu
               210                 215                 220
   GAG AAG AAA AAT GAA ACA AAG GGT TCC ATG CTA AAA CTT ACA AAA CAT    779
   Glu Lys Lys Asn Glu Thr Lys Gly Ser Met Leu Lys Leu Thr Lys His
           225                 230                 235
   CAA GTT GAA ATG TTG AGA AAA AAG GCG AAC CAA GGT AAT CAA GGG CGT    827
   Gln Val Glu Met Leu Arg Lys Lys Ala Asn Gln Gly Asn Gln Gly Arg
       240                 245                 250
   AGT TAC ACA CGT TAT GAA GTT GTG ACT GCA CAT ATA TGG AGA TGT GCA    875
   Ser Tyr Thr Arg Tyr Glu Val Val Thr Ala His Ile Trp Arg Cys Ala
   255                 260                 265                 270
   TGC AAG GCA AGA GGT CAT AAA TTT GAG CAG CCT ACT AAT TTA TGC ATT    923
   Cys Lys Ala Arg Gly His Lys Phe Glu Gln Pro Thr Asn Leu Cys Ile
                   275                 280                 285
   TGT GTT AAC ATA CGC AAT ATA ATG CAA CCA CCT TTG CCT AAA TCC TAT    971
   Cys Val Asn Ile Arg Asn Ile Met Gln Pro Pro Leu Pro Lys Ser Tyr
               290                 295                 300
   TTT GGC AAT GCC ATA GTT GAT GTT ATT GCC AAT GGC GTC TCG GGT GAC   1019
   Phe Gly Asn Ala Ile Val Asp Val Ile Ala Asn Gly Val Ser Gly Asp
           305                 310                 315
   ATT ACC TCG AGG CCA TTG GAG TAT GTT GCT CGA AGG GTG CGA GCA GCC   1067
   Ile Thr Ser Arg Pro Leu Glu Tyr Val Ala Arg Arg Val Arg Ala Ala
       320                 325                 330
   ATT AAA ATG GTG ACG AGT GAT TAC GCA AAC TCG ACG ATT GAT TTC TTA   1115
   Ile Lys Met Val Thr Ser Asp Tyr Ala Asn Ser Thr Ile Asp Phe Leu
   335                 340                 345                 350
   AAA AAC CAG GAG GAT TTG TCA AAA TAT CAA GAT ATT CAT GCA TTT AGA   1163
   Lys Asn Gln Glu Asp Leu Ser Lys Tyr Gln Asp Ile His Ala Phe Arg
                   355                     360                     365
   AGC AAG GAA GGT CCT TTT TAT GGA AAC CCT AAT CTT GGG GTT ATA AGT  1211
   Ser Lys Glu Gly Pro Phe Tyr Gly Asn Pro Asn Leu Gly Val Ile Ser
               370                     375                     380
   TGG ATA AGT TTG CCA TTA TTA GGA TTG GAT TTT GGG TGG GGA AAA GAG  1259
   Trp Ile Ser Leu Pro Leu Leu Gly Leu Asp Phe Gly Trp Gly Lys Glu
           385                     390                     395
   ATA CAT ATG AGC CCT GGA ACT CAT GAA TAT GAT GGT GAT TGT GTG ATA  1307
   Ile His Met Ser Pro Gly Thr His Glu Tyr Asp Gly Asp Cys Val Ile
       400                     405                     410
   CTT CCA GGA AAA GAA GGG GAT GGA TCT TTG ACT GTT GCA ATC ATT CTT  1355
   Leu Pro Gly Lys Glu Gly Asp Gly Ser Leu Thr Val Ala Ile Ile Leu
   415                     420                     425                 430
   CAA GCT GTT CAT GTG GAT GCT TTC AAG AAC TTC TTC TAT GAA GAA ATT  1403
   Gln Ala Val His Val Asp Ala Phe Lys Asn Phe Phe Tyr Glu Glu Ile
                   435                     440                     445
   GAA TGT TGAAAAACAT AAGTGTTTTA TGAGAAGAAA GGAAACAAAT TAAGAACATG   1459
   Glu Cys
   TAGCTTTTCC TAAATTGACA TTGTTAGTCA TGGTCTAAGC AAAATAAACT CTTTATCTAC 1519
   ACATTATTTC AATATATTTT CCT                                        1542
(2) INFORMATION FOR SEQ ID NO:2:
   (i) SEQUENCE CHARACTERISTICS:
      (A) LENGTH: 448 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Lys Val Ser Leu Lys His His Trp Val Val Lys Pro Ala Glu Ala
   1 5 10 15
   Thr Trp Asn Gly Thr Val Ser Leu Ser Glu Cys Asp Gln Thr Phe Ala
   20 25 30
   Val Thr His Val Pro Thr Ile Tyr Tyr Tyr Arg Phe Cys His Asp Cys
   35 40 45
   Leu Pro Ser Thr Asp Asn Ile Ile Lys Thr Leu Arg Thr Ser Leu Ser
   50 55 60
   Lys Ala Leu Val His Phe Tyr Pro Leu Ser Gly Arg Leu Arg Trp Ile
   65 70 75 80
   Ala Gly Ser Arg Leu Glu Leu Asp Cys Asn Ala Ser Gly Ile Val Leu
   85 90 95
   Met Glu Ala Glu Thr Glu Ala Lys Leu Asp Asp Leu Gly Asp Phe Ser
   100 105 110
   Pro Ser Pro Asp Leu Asn Ser Leu Phe Pro Arg Val Asp Tyr Thr Ile
   115 120 125
   Pro Ile Asp Glu Leu Pro Leu Leu Phe Val Gln Leu Thr Lys Phe Gln
   130 135 140
   Cys Gly Gly Ile Ala Leu Ser Phe Ala Ile Ser His Ala Val Val Asp
   145 150 155 160
   Gly Gln Ser Ala Leu Tyr Phe Leu Thr Glu Trp Ala Ser Leu Ala Arg
   165 170 175
   Gly Glu Pro Leu Gly Asn Glu Pro Phe His Asp Arg Lys Phe Leu Arg
   180 185 190
   Ala Gly Glu Pro Pro Ile Ala Tyr Pro Thr Phe Glu His Leu Gln Phe
   195 200 205
   Asn Pro Pro Pro Leu Leu Leu Gly Gln Ser Ser Ser Glu Glu Glu Lys
   210 215 220
   Lys Asn Glu Thr Lys Gly Ser Met Leu Lys Leu Thr Lys His Gln Val
   225 230 235 240
   Glu Met Leu Arg Lys Lys Ala Asn Gln Gly Asn Gln Gly Arg Ser Tyr
   245 250 255
   Thr Arg Tyr Glu Val Val Thr Ala His Ile Trp Arg Cys Ala Cys Lys
   260 265 270
   Ala Arg Gly His Lys Phe Glu Gln Pro Thr Asn Leu Cys Ile Cys Val
   275 280 285
   Asn Ile Arg Asn Ile Met Gln Pro Pro Leu Pro Lys Ser Tyr Phe Gly
   290 295 300
   Asn Ala Ile Val Asp Val Ile Ala Asn Gly Val Ser Gly Asp Ile Thr
   305 310 315 320
   Ser Arg Pro Leu Glu Tyr Val Ala Arg Arg Val Arg Ala Ala Ile Lys
   325 330 335
   Met Val Thr Ser Asp Tyr Ala Asn Ser Thr Ile Asp Phe Leu Lys Asn
   340 345 350
   Gln Glu Asp Leu Ser Lys Tyr Gln Asp Ile His Ala Phe Arg Ser Lys
   355 360 365
   Glu Gly Pro Phe Tyr Gly Asn Pro Asn Leu Gly Val Ile Ser Trp Ile
   370 375 380
   Ser Leu Pro Leu Leu Gly Leu Asp Phe Gly Trp Gly Lys Glu Ile His
   385 390 395 400
   Met Ser Pro Gly Thr His Glu Tyr Asp Gly Asp Cys Val Ile Leu Pro
   405 410 415
   Gly Lys Glu Gly Asp Gly Ser Leu Thr Val Ala Ile Ile Leu Gln Ala
   420 425 430
   Val His Val Asp Ala Phe Lys Asn Phe Phe Tyr Glu Glu Ile Glu Cys
   435 440 445
(2) INFORMATION FOR SEQ ID NO:3:
   (i) SEQUENCE CHARACTERISTICS:
      (A) LENGTH: 552 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant43D
   (ix) FEATURE:
      (A) NAME/KEY: CDS
      (B) LOCATION: 41..397
   (xi) SEQUENCE DESCRIPTION: SEQ ID NO:3:
   CTTACATTTC TTCAATAGTT TAGTCCATAA AGCAATAGAT ATG GCT CGG TTT CTT   55
                                               Met Ala Arg Phe Leu
                                                 1               5
   GTG TTC CTT GCT TTA GCC CTT GTA ATA ATT TCA AAG AAG GGC GCG TTG  103
   Val Phe Leu Ala Leu Ala Leu Val Ile Ile Ser Lys Lys Gly Ala Leu
                    10                  15                  20
   GGT GCT CCT CCT TCC TGT CCA ACA GTT ACA ACG CAG CTG GCT CCT TGT  151
   Gly Ala Pro Pro Ser Cys Pro Thr Val Thr Thr Gln Leu Ala Pro Cys
                25                  30                  35
   CTA TCG TAC ATT CAA GGT GGA GGT GAT CCA TCT GTA CCT TGC TGC ACT  199
   Leu Ser Tyr Ile Gln Gly Gly Gly Asp Pro Ser Val Pro Cys Cys Thr
            40                  45                  50
   GGT ATA AAT AAC ATA TAT GAA CTT GCT AAA ACC AAA GAA GAC CGA GTC  247
   Gly Ile Asn Asn Ile Tyr Glu Leu Ala Lys Thr Lys Glu Asp Arg Val
        55                  60                  65
   GCT ATC TGC AAC TGC TTA AAA ACC GCA TTT ACT CAT GCT GGA AAT GTC  295
   Ala Ile Cys Asn Cys Leu Lys Thr Ala Phe Thr His Ala Gly Asn Val
    70                  75                  80                  85
   AAT CCC ACT CTC GTA GCT CAA CTC CCC AAG AAA TGT GGC ATT TCT TTT  343
   Asn Pro Thr Leu Val Ala Gln Leu Pro Lys Lys Cys Gly Ile Ser Phe
                       90                  95                  100
   AAT ATG CCT CCT ATT GAT AAA AAC TAC GAC TGT AAC ACG ATT TCT ATG  391
   Asn Met Pro Pro Ile Asp Lys Asn Tyr Asp Cys Asn Thr Ile Ser Met
               105                 110                 115
   TAC TGATGAATGG GTAGTGAATC TCGGAAGCTG CTCAAATTTA TGAATAAAAC       444
   Tyr
   ATATATAGAT GTTCATCTCA TGTCTGAAAT CTGAAAGCAA TTTGATCCAC TGTAAACTTC 504
   AAATGTATGC AGACGGTTAA ATGTTGAATT ATGATATATA TAAATTTG              552
(2) INFORMATION FOR SEQ ID NO:4:
   (i) SEQUENCE CHARACTERISTICS:
      (A) LENGTH: 118 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Ala Arg Phe Leu Val Phe Leu Ala Leu Ala Leu Val Ile Ile Ser
    1               5                  10                  15
   Lys Lys Gly Ala Leu Gly Ala Pro Pro Ser Cys Pro Thr Val Thr Thr
                20                  25                  30
   Gln Leu Ala Pro Cys Leu Ser Tyr Ile Gln Gly Gly Gly Asp Pro Ser
            35                  40                  45
   Val Pro Cys Cys Thr Gly Ile Asn Asn Ile Tyr Glu Leu Ala Lys Thr
   50 55 60
   Lys Glu Asp Arg Val Ala Ile Cys Asn Cys Leu Lys Thr Ala Phe Thr
   65 70 75 80
   His Ala Gly Asn Val Asn Pro Thr Leu Val Ala Gln Leu Pro Lys Lys
   85 90 95
   Cys Gly Ile Ser Phe Asn Met Pro Pro Ile Asp Lys Asn Tyr Asp Cys
   100 105 110
   Asn Thr Ile Ser Met Tyr
   115
(2) INFORMATION FOR SEQ ID NO:5:
   (i) SEQUENCE CHARACTERISTICS:
      (A) LENGTH: 612 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant9
   (ix) FEATURE:
      (A) NAME/KEY: CDS
      (B) LOCATION: 344..442
   (xi) SEQUENCE DESCRIPTION: SEQ. ID NO:5:
   TGTCAGAGAG GGTGATGTTT TAACATTGTT AGAGTCTGAC AGGTCCCTTG ACATTTCTCA   60
   TGATAAACCT GTTCTGGTCA TCATGAGAAA TGTCTAGCCT CTCTCTCAGA CTCTAACAAT  120
   GTTAAAACAT CACCCTCTCT GACAGGTCCC TTGACATTTC TCATGATAAA CCTGTTCTGG  180
   TCATCAAGAA ACTTGACTCT CACCTGAGTT ACCTGTCCTC TGGACCCAGT ACGGCCCATG  240
   ACTTTCACCA CAATAGCATG CTTGGTCGCA GATTCCATCC TTGAGAGGAG CAGACGAGCG  300
   AGCACAAAGC GCAAATTGCT ATGACGGCCG AATAGGAGAA AAA ATG CCT TCC CTC    355
                                                   Met Pro Ser Leu
                                                   1
   TCA GTG CAA TCT TCC TCC CCT CTC TTG TGC GGC AAA CTG AGT TTG ATG    403
   Ser Val Gln Ser Ser Ser Pro Leu Leu Cys Gly Lys Leu Ser Leu Met
     5                  10                  15                  20
   GGG TCC GTG CCT ACC AGT TCC CAG TCA CTG GGC GAA TAATATCATA         449
   Gly Ser Val Pro Thr Ser Ser Gln Ser Leu Gly Glu
                   25                  30
   GTTCTAAAAT CAATAAATTT ACTTTGTCCC TTCTATCTTT TTTTTCTTCT TTTTCATTGG  509
   TGCTCTTTAT GCTAATGTCC TCACTCCTCT GTTCTATCAC AGAGCAAGGT CAGGAAAGAG  569
   TTTGTATTGT CATATGAAAT CAATAAAACA AACTGTTTAC CCG                    612
(2) INFORMATION FOR SEQ ID NO:6:
   (i) SEQUENCE CHARACTERISTICS:
      (A) LENGTH: 32 amino acids
      (B) TYPE:amino acid
      (D) TOPOLOGY: linear
   (ii) MOLECULE TYPE: protein
   (xi) SEQUENCE DESCRIPTION: SEQ ID NO:6:
   Met Pro Ser Leu Ser Val Gln Ser Ser Ser Pro Leu Leu Cys Gly Lys
   1 5 10 15
   Leu Ser Leu Met Gly Ser Val Pro Thr Ser Ser Gln Ser Leu Gly Glu
   20 25 30
(2) INFORMATION FOR SEQ ID NO:7:
   (i) SEQUENCE CHARACTERISTICS:
      (A) LENGTH: 96 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant52
   (ix) FEATURE:
      (A) NAME/KEY: CDS
      (B) LOCATION: 3..95
   (xi) SEQUENCE DESCRIPTION: SEQ ID NO:7:
   CC CAT AAC TGC CTT AAT TGC AAT TCT AAA AGG CAA CAA GAT TCT TAC 47
   His Asn Cys Leu Asn Cys Asn Ser Lys Arg Gln Gln Asp Ser Tyr
   1 5 10 15
   TTC TTC ACT GAT CCA ATG AAA GCA CAA TCA ATA GTA GGA ACT GTC ACC 95
   Phe Phe Thr Asp Pro Met Lys Ala Gln Ser Ile Val Gly Thr Val Thr
   20 25 30
   C 96
(2) INFORMATION FOR SEQ ID NO:8:
   (i) SEQUENCE CHARACTERISTICS:
      (A) LENGTH: 31 amino acids
      (B) TYPE: amino acid
      (D) TOPOLOGY: linear
   (ii) MOLECULE TYPE: protein
   (xi) SEQUENCE DESCRIPTION: SEQ ID NO:8:
   His Asn Cys Leu Asn Cys Asn Ser Lys Arg Gln Gln Asp Ser Tyr Phe
    1               5                  10                  15
   Phe Thr Asp Pro Met Lys Ala Gln Ser Ile Val Gly Thr Val Thr
               20                  25                  30
(2) INFORMATION FOR SEQ ID NO:9:
   (i) SEQUENCE CHARACTERISTICS:
      (A) LENGTH: 1201 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant59
   (ix) FEATURE:
      (A) NAME/KEY: CDS
      (B) LOCATION: 1..1119
   (xi) SEQUENCE DESCRIPTION: SEQ ID NO:9:
   ATC TTT AGT AGC CAA ATA TGG ACT CAA CCT AAT TCT GAA ATG AAT AAT   48
   Ile Phe Ser Ser Gln Ile Trp Thr Gln Pro Asn Ser Glu Met Asn Asn
    1               5                  10                  15
   GAT CTT GTG ATC CCC GCC ATT TTC AAC CAT GAG AAG CTT AGG ACC ATT   96
   Asp Leu Val Ile Pro Ala Ile Phe Asn His Glu Lys Leu Arg Thr Ile
               20                  25                  30
   TCA CGT GAA TGC GAT CCC AAG CGT AAA CTA GCC GAA AGC AAT TCA GGA  144
   Ser Arg Glu Cys Asp Pro Lys Arg Lys Leu Ala Glu Ser Asn Ser Gly
           35                  40                  45
   GAC ATC ATG GGA GAA GTT AAG AAG ACT CAT CAA GCT ATT CAA TCA CTT  192
   Asp Ile Met Gly Glu Val Lys Lys Thr His Gln Ala Ile Gln Ser Leu
       50                  55                  60
   GAT AAA AGT ATG TCA ACA TTG GAG AAT GAA TTG GCA ATA GCT CGG ACA  240
   Asp Lys Ser Met Ser Thr Leu Glu Asn Glu Leu Ala Ile Ala Arg Thr
    65                  70                  75                  80
   AGG CAA ACA ATC AGT CAC AAT GCA AAG GAA AAT AGG GCT TCA AAT CAC  288
   Arg Gln Thr Ile Ser His Asn Ala Lys Glu Asn Arg Ala Ser Asn His
                    85                  90                  95
   ACC ACA CCG AAT AAA GCA TTC ATC GTG GTG GGA ATT AAT ACC GCA TTC  336
   Thr Thr Pro Asn Lys Ala Phe Ile Val Val Gly Ile Asn Thr Ala Phe
               100                 105                 110
   AGC AGC AGA AAA AGA CGC GAT TCT CTT AGA GAA ACT TGG ATG CCT AAA  384
   Ser Ser Arg Lys Arg Arg Asp Ser Leu Arg Glu Thr Trp Met Pro Lys
           115                 120                 125
   GGG GAT AAG CTA AGG AAG CTA GAG AAA GAG AAG GGA ATC GTG ATA CGG  432
   Gly Asp Lys Leu Arg Lys Leu Glu Lys Glu Lys Gly Ile Val Ile Arg
       130                 135                 140
   TTT GTG ATA GGA CAC AGT GCT ACA CGA GGA GGA GTT CTT GAT CGT GCC  480
   Phe Val Ile Gly His Ser Ala Thr Arg Gly Gly Val Leu Asp Arg Ala
   145                 150                 155                 160
   ATT GAT AGT GAG GAT GCT CAG TAC AAG GAT TTC CTT CGA CTT GAC CAC  528
   Ile Asp Ser Glu Asp Ala Gln Tyr Lys Asp Phe Leu Arg Leu Asp His
                   165                 170                 175
   GTT GAG GGT TAT CAT GAG CTG TCC ACC AAG ACA AGA TTG TAT TTC TCT  576
   Val Glu Gly Tyr His Glu Leu Ser Thr Lys Thr Arg Leu Tyr Phe Ser
               180                 185                 190
   AAA GCT GTC TCC ATT TGG GAC GCT GAC TTC TAC GTT AAA GTG GAC GAT  624
   Lys Ala Val Ser Ile Trp Asp Ala Asp Phe Tyr Val Lys Val Asp Asp
           195                 200                 205
   GAT GTC CAT CTC AAC TTA GGT ATG CTT GCG AAC ACA TTA GCA AAA TAC  672
   Asp Val His Leu Asn Leu Gly Met Leu Ala Asn Thr Leu Ala Lys Tyr
       210                 215                 220
   AAA TCC AAA CCA AGA GTC TAC ATT GGA TGC ATG AAA TCA GGG CCA GTT  720
   Lys Ser Lys Pro Arg Val Tyr Ile Gly Cys Met Lys Ser Gly Pro Val
   225                 230                 235                 240
   CTT TCC CAA AAA GGA GTA AGG TAT TAT GAG CCC GAG TAT TGG AAA TTT  768
   Leu Ser Gln Lys Gly Val Arg Tyr Tyr Glu Pro Glu Tyr Trp Lys Phe
                   245                 250                 255
   GGA GAA GAA GGA AAC AAG TAT TTC AGG CAT GCC ACG GGT CAA ATA TAT  816
   Gly Glu Glu Gly Asn Lys Tyr Phe Arg His Ala Thr Gly Gln Ile Tyr
               260                 265                 270
   GGC ATC TCT AGA GAC CTT GCT TCA TAT ATC TCC ATC AAC TCG GGA ATA  864
   Gly Ile Ser Arg Asp Leu Ala Ser Tyr Ile Ser Ile Asn Ser Gly Ile
           275                 280                 285
   TTA CAT AGA TAT GCA AAT GAA GAC GTA TCA TTG GGA TCA TGG TTA ATT  912
   Leu His Arg Tyr Ala Asn Glu Asp Val Ser Leu Gly Ser Trp Leu Ile
       290                 295                 300
   GGG TTG GAA GTA GAG CAT GTG GAT GAG CGT TCA ATG TGC TGT GGA ACA  960
   Gly Leu Glu Val Glu His Val Asp Glu Arg Ser Met Cys Cys Gly Thr
   305                 310                 315                 320
   CCT CCA GAT TGT GAG TGG AAA GCC AAA GGA GGA AAT ATA TGT GTG GCA  1008
   Pro Pro Asp Cys Glu Trp Lys Ala Lys Gly Gly Asn Ile Cys Val Ala
                   325                 330                 335
   TCA TTT GAT TGG TCA TGC AGT GGG ATA TGC AAG TCG GTA GAG AGG ATG  1056
   Ser Phe Asp Trp Ser Cys Ser Gly Ile Cys Lys Ser Val Glu Arg Met
               340                 345                 350
   AAA GAT GTG CAC CAC TCA TGC GGC GAA GGT GAC GCA GCT CTT TGG AAT   1104
   Lys Asp Val His His Ser Cys Gly Glu Gly Asp Ala Ala Leu Trp Asn
           355                 360                 365
   GTT CCT CTC TCA TGAGATTTAT TGGAGAGAAC TTAATTAATT ATCCACATAG      1156
   Val Pro Leu Ser
       370
   TATTTCCTTT CGATTAATTA ATAATTTACT TGCGCAATGC AATTC               1201
(2) INFORMATION FOR SEQ ID NO:10:
   (i) SEQUENCE CHARACTERISTICS:
      (A) LENGTH: 372 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Ile Phe Ser Ser Gln Ile Trp Thr Gln Pro Asn Ser Glu Met Asn Asn
    1               5                  10                  15
   Asp Leu Val Ile Pro Ala Ile Phe Asn His Glu Lys Leu Arg Thr Ile
               20                  25                  30
   Ser Arg Glu Cys Asp Pro Lys Arg Lys Leu Ala Glu Ser Asn Ser Gly
           35                  40                  45
   Asp Ile Met Gly Glu Val Lys Lys Thr His Gln Ala Ile Gln Ser Leu
       50                  55                  60
   Asp Lys Ser Met Ser Thr Leu Glu Asn Glu Leu Ala Ile Ala Arg Thr
   65                  70                  75                  80
   Arg Gln Thr Ile Ser His Asn Ala Lys Glu Asn Arg Ala Ser Asn His
   85 90 95
   Thr Thr Pro Asn Lys Ala Phe Ile Val Val Gly Ile Asn Thr Ala Phe
   100 105 110
   Ser Ser Arg Lys Arg Arg Asp Ser Leu Arg Glu Thr Trp Met Pro Lys
   115 120 125
   Gly Asp Lys Leu Arg Lys Leu Glu Lys Glu Lys Gly Ile Val Ile Arg
   130 135 140
   Phe Val Ile Gly His Ser Ala Thr Arg Gly Gly Val Leu Asp Arg Ala
   145 150 155 160
   Ile Asp Ser Glu Asp Ala Gln Tyr Lys Asp Phe Leu Arg Leu Asp His
   165 170 175
   Val Glu Gly Tyr His Glu Leu Ser Thr Lys Thr Arg Leu Tyr Phe Ser
   180 185 190
   Lys Ala Val Ser Ile Trp Asp Ala Asp Phe Tyr Val Lys Val Asp Asp
   195 200 205
   Asp Val His Leu Asn Leu Gly Met Leu Ala Asn Thr Leu Ala Lys Tyr
   210 215 220
   Lys Ser Lys Pro Arg Val Tyr Ile Gly Cys Met Lys Ser Gly Pro Val
   225 230 235 240
   Leu Ser Gln Lys Gly Val Arg Tyr Tyr Glu Pro Glu Tyr Trp Lys Phe
   245 250 255
   Gly Glu Glu Gly Asn Lys Tyr Phe Arg His Ala Thr Gly Gln Ile Tyr
   260 265 270
   Gly Ile Ser Arg Asp Leu Ala Ser Tyr Ile Ser Ile Asn Ser Gly Ile
   275 280 285
   Leu His Arg Tyr Ala Asn Glu Asp Val Ser Leu Gly Ser Trp Leu Ile
   290 295 300
   Gly Leu Glu Val Glu His Val Asp Glu Arg Ser Met Cys Cys Gly Thr
   305 310 315 320
   Pro Pro Asp Cys Glu Trp Lys Ala Lys Gly Gly Asn Ile Cys Val Ala
   325 330 335
   Ser Phe Asp Trp Ser Cys Ser Gly Ile Cys Lys Ser Val Glu Arg Met
   340 345 350
   Lys Asp Val His His Ser Cys Gly Glu Gly Asp Ala Ala Leu Trp Asn
   355 360 365
   Val Pro Leu Ser
   370
(2) INFORMATION FOR SEQ ID NO:11:
   (i) SEQUENCE CHARACTERISTICS:
      (A) LENGTH: 952 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant66
   (ix) FEATURE:
      (A) NAME/KEY: CDS
      (B) LOCATION: 1..711
   (xi) SEQUENCE DESCRIPTION: SEQ ID NO:11:
   ATC ATG ACC ATC TCT AAG GAC AGG GTG AAG CCA TCC CCT CTG CCC GAC    48
   Ile Met Thr Ile Ser Lys Asp Arg Val Lys Pro Ser Pro Leu Pro Asp
     1               5                  10                  15
   TCG TGG AAG CTC AAC GAA ATC TTT GCC ACT GGA ATC GTC CTC GGA ACC    96
   Ser Trp Lys Leu Asn Glu Ile Phe Ala Thr Gly Ile Val Leu Gly Thr
                20                  25                  30
   TAT CAA GCT ATT ATG ACT GTG GTG TTC TTC TAT CTT GCA GCT GAC ACT   144
   Tyr Gln Ala Ile Met Thr Val Val Phe Phe Tyr Leu Ala Ala Asp Thr
            35                  40                  45
   GAC TTC TTT ACA GAG AAA TTC AAC GTT AAA TCA ATC AGG GAT AAT CCC   192
   Asp Phe Phe Thr Glu Lys Phe Asn Val Lys Ser Ile Arg Asp Asn Pro
        50                  55                  60
   TAC GAG CTT ACA GCT GCT GTA TAC CTT CAA GTG AGC ATC ATC AGC CAA   240
   Tyr Glu Leu Thr Ala Ala Val Tyr Leu Gln Val Ser Ile Ile Ser Gln
    65                  70                  75                  80
   GCT CTT ATC TTT GTG ACA AGA TCA AGA AGC TGG TCA TTT TTG GAA CGC   288
   Ala Leu Ile Phe Val Thr Arg Ser Arg Ser Trp Ser Phe Leu Glu Arg
                    85                  90                  95
   CCG GGT TTC TTG CTT GTC ACT GCT TTC CTC TTA GCC CAA TTT GTG GCT  336
   Pro Gly Phe Leu Leu Val Thr Ala Phe Leu Leu Ala Gln Phe Val Ala
               100                 105                 110
   ACA TTA ATC GCT GTC TAC GCC AAC TGG AAG TTT GCT AGG ATC CAT GGA  384
   Thr Leu Ile Ala Val Tyr Ala Asn Trp Lys Phe Ala Arg Ile His Gly
           115                 120                 125
   ATT GGT TGG GGA TGG GCA GGA ATC ATC TGG ATC TAC ACA ATT ATC ACC  432
   Ile Gly Trp Gly Trp Ala Gly Ile Ile Trp Ile Tyr Thr Ile Ile Thr
       130                 135                 140
   TAT ATC CCT CTT GAT ATT CTC AAA TTC ATC AGT CGT TAC ACG TTG AGT  480
   Tyr Ile Pro Leu Asp Ile Leu Lys Phe Ile Ser Arg Tyr Thr Leu Ser
   145                 150                 155                 160
   GGT GAG GCC TGG AAT TCA ATG ATC CAA AAT AAG ACT GCT TTC ACA ACC  528
   Gly Glu Ala Trp Asn Ser Met Ile Gln Asn Lys Thr Ala Phe Thr Thr
                   165                 170                 175
   AAG AAG GAT TAT GGA AAA GGT GAG AGG GAA GCA CAA TGG GCT GTG GCG  576
   Lys Lys Asp Tyr Gly Lys Gly Glu Arg Glu Ala Gln Trp Ala Val Ala
               180                 185                 190
   CAA CGA ACA CTA CAC GGT CTC CAG ACT GCT GAA AGC AAT GGC CTA TTC  624
   Gln Arg Thr Leu His Gly Leu Gln Thr Ala Glu Ser Asn Gly Leu Phe
           195                 200                 205
   CAT GAC AAG AAC TAC AGA GAA TTG AAT GAG ATT GCT GAA CAG GCT AAA  672
   His Asp Lys Asn Tyr Arg Glu Leu Asn Glu Ile Ala Glu Gln Ala Lys
       210                 215                 220
   CGT CGC GCT GAA GTT GCA AAA TAT ACA CAT GAG CCA TGAAAATAAC       718
   Arg Arg Ala Glu Val Ala Lys Tyr Thr His Glu Pro
   225                 230                 235
   TTGATTATCT CAATAACCAT GTTGCAAGAT AGGGGAATAT TAGACTCTCA AGGGACATGT 778
   TAAATCTATG TAGTCTAAGT TAAAGGGCAT TTTTGCAGCT ATTTATCAAG AATGTATCTC 838
   AATGTTGGAT GAAATCCAAT ATTGGTGAAC TACAAAGGCT AGCTGCTAAT CAAAACTATT 898
   AAACTAGTAG TTATATACAT AAAGAAAATT TACTATAGCA AAAAAAAAAA AAAA       952
(2) INFORMATION FOR SEQ ID NO:12:
   (i) SEQUENCE CHARACTERISTICS:
      (A) LENGTH: 236 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Ile Met Thr Ile Ser Lys Asp Arg Val Lys Pro Ser Pro Leu Pro Asp
    1               5                  10                  15
   Ser Trp Lys Leu Asn Glu Ile Phe Ala Thr Gly Ile Val Leu Gly Thr
               20                  25                  30
   Tyr Gln Ala Ile Met Thr Val Val Phe Phe Tyr Leu Ala Ala Asp Thr
           35                  40                  45
   Asp Phe Phe Thr Glu Lys Phe Asn Val Lys Ser Ile Arg Asp Asn Pro
       50                  55                  60
   Tyr Glu Leu Thr Ala Ala Val Tyr Leu Gln Val Ser Ile Ile Ser Gln
   65                  70                  75                  80
   Ala Leu Ile Phe Val Thr Arg Ser Arg Ser Trp Ser Phe Leu Glu Arg
   85 90 95
   Pro Gly Phe Leu Leu Val Thr Ala Phe Leu Leu Ala Gln Phe Val Ala
   100 105 110
   Thr Leu Ile Ala Val Tyr Ala Asn Trp Lys Phe Ala Arg Ile His Gly
   115 120 125
   Ile Gly Trp Gly Trp Ala Gly Ile Ile Trp Ile Tyr Thr Ile Ile Thr
   130 135 140
   Tyr Ile Pro Leu Asp Ile Leu Lys Phe Ile Ser Arg Tyr Thr Leu Ser
   145 150 155 160
   Gly Glu Ala Trp Asn Ser Met Ile Gln Asn Lys Thr Ala Phe Thr Thr
   165 170 175
   Lys Lys Asp Tyr Gly Lys Gly Glu Arg Glu Ala Gln Trp Ala Val Ala
   180 185 190
   Gln Arg Thr Leu His Gly Leu Gln Thr Ala Glu Ser Asn Gly Leu Phe
   195 200 205
   His Asp Lys Asn Tyr Arg Glu Leu Asn Glu Ile Ala Glu Gln Ala Lys
   210 215 220
   Arg Arg Ala Glu Val Ala Lys Tyr Thr His Glu Pro
   225 230 235
(2) INFORMATION FOR SEQ ID NO:13:
   (i) SEQUENCE CHARACTERISTICS:
      (A) LENGTH: 305 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (vi) ORIGINAL SOURCE:
      (A) ORGANISM: Nicotiana tabacum
      (C) INDIVIDUAL ISOLATE: Ant67
   (xi) SEQUENCE DESCRIPTION: SEQ ID NO:13:
   ATTAAACTCT TGTTGTGTTT CCCTAGATTC CCAAGTTCTT TTTAGCTCCA TGCTTCTTGT 60
   CCTCATGGCA TCCTGCTCTC TGTAAAAATT GAATCTTTTT ATGTTTTACT TCCATTCTTG 120
   AATTTCATCC CTTTTGTTTG CTTCAATTGT TGCTTCTACC TTAATCATTT ATGTATTCCA 180
   TGTTGTGGGT TTTGCTTCTT CATTTTAAGT TTAACTCCTG TGCCCTAAGA TAATTTTTTT 240
   TAATGTTTTT CTTCCATTCT TGATTTTCTT TTTCTGTGCA TTAGGCCTTT TTGTATATTT 300
   CTTGT 305
(2) INFORMATION FOR SEQ ID NO:14:
   (i) SEQUENCE CHARACTERISTICS:
      (A) LENGTH: 445 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant68
   (ix) FEATURE:
      (A) NAME/KEY: CDS
      (B) LOCATION: 2..445
   (xi) SEQUENCE DESCRIPTION: SEQ ID NO:14:
   A GTT GGT GGC GGT GGC AGT GGC GGA GGT GGA GCC TAT GGT AGC GGG    46
     Val Gly Gly Gly Gly Ser Gly Gly Gly Gly Ala Tyr Gly Ser Gly
      1               5                  10                  15
   TGT GGT GAA AAT GGC TGT AAT TAC CCG CCC GTT GTA CCT GGA CCT CCA  94
   Cys Gly Glu Asn Gly Cys Asn Tyr Pro Pro Val Val Pro Gly Pro Pro
                   20                  25                  30
   CAA ACA GGC GAA AAC CCT TAT TGC ATG CCT GGT TGT GGC GTA GGT GGT  142
   Gln Thr Gly Glu Asn Pro Tyr Cys Met Pro Gly Cys Gly Val Gly Gly
               35                  40                  45
   GGT GGG GTA GGC GGC AGT AAT GGC GGA AGT GGC GGT GGA GGA GGC GGT  190
   Gly Gly Val Gly Gly Ser Asn Gly Gly Ser Gly Gly Gly Gly Gly Gly
           50                  55                  60
   GGT GGT GGC GGA GGT GGA GGT GGA GGA TAT GGT AGT GGT TAT GGT GAA  238
   Gly Gly Gly Gly Gly Gly Gly Gly Gly Tyr Gly Ser Gly Tyr Gly Glu
       65                  70                  75
   AAT GGA AAT TGT AAT TAC CCA CCC GTT ATA CCT GGA CCC CCA CAA ACA   286
   Asn Gly Asn Cys Asn Tyr Pro Pro Val Ile Pro Gly Pro Pro Gln Thr
    80                 85                  90                  95
   ATT GGA CCT ATA TGC AAT TGT CCA ATA ACT CAA CCA ACA TTC CCA TTT   334
   Ile Gly Pro Ile Cys Asn Cys Pro Ile Thr Gln Pro Thr Phe Pro Phe
                   100                 105                 110
   CGT TGT CCA TAT GGA TGT CAG CCA CCA CCT AGT TAT GGC TGC CCA AAT   382
   Arg Cys Pro Tyr Gly Cys Gln Pro Pro Pro Ser Tyr Gly Cys Pro Asn
               115                 120                 125
   GGA AAT TCC AGA CTA ACT CAT GAC AAG GAA AAA CAG AAT CAT CAG CCC   430
   Gly Asn Ser Arg Leu Thr His Asp Lys Glu Lys Gln Asn His Gln Pro
           130                 135                 140
   AAG ACT ACT GCT TCG                                               445
   Lys Thr Thr Ala Ser
       145
(2) INFORMATION FOR SEQ ID NO:15:
   (i) SEQUENCE CHARACTERISTICS:
      (A) LENGTH: 148 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Val Gly Gly Gly Gly Ser Gly Gly Gly Gly Ala Tyr Gly Ser Gly Cys
    1               5                  10                  15
   Gly Glu Asn Gly Cys Asn Tyr Pro Pro Val Val Pro Gly Pro Pro Gln
                   20                  25                  30
   Thr Gly Glu Asn Pro Tyr Cys Met Pro Gly Cys Gly Val Gly Gly Gly
               35                  40                  45
   Gly Val Gly Gly Ser Asn Gly Gly Ser Gly Gly Gly Gly Gly Gly Gly
           50                  55                  60
   Gly Gly Gly Gly Gly Gly Gly Gly Tyr Gly Ser Gly Tyr Gly Glu Asn
   65                  70                  75                  80
   Gly Asn Cys Asn Tyr Pro Pro Val Ile Pro Gly Pro Pro Gln Thr Ile
                   85                  90                  95
   Gly Pro Ile Cys Asn Cys Pro Ile Thr Gln Pro Thr Phe Pro Phe Arg
               100                 105                 110
   Cys Pro Tyr Gly Cys Gln Pro Pro Pro Ser Tyr Gly Cys Pro Asn Gly
           115                 120                 125
   Asn Ser Arg Leu Thr His Asp Lys Glu Lys Gln Asn His Gln Pro Lys
       130                 135                 140
   Thr Thr Ala Ser
   145
(2) INFORMATION FOR SEQ ID NO:16:
   (i) SEQUENCE CHARACTERISTICS:
      (A) LENGTH: 3706 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant32 genomic clone
   (vii) IMMEDIATE SOURCE:
      (B) CLONE: pCIB950
   (ix) FEATURE:
      (A) NAME/KEY: TATA_signal
      (B) LOCATION: 1971..1975
   (ix) FEATURE:
      (A) NAME/KEY: CDS
      (B) LOCATION: 2076..3422
   (ix) FEATURE:
      (A) NAME/KEY: misc_feature
      (B) LOCATION: 2009
      (D) OTHER INFORMATION: /note= "Putative transcription start site"
   (xi) SEQUENCE DESCRIPTION: SEQ ID NO:16:
   CTGCAGTAAG GGGGATATTC AGAGACTCAA CTTAATCAAT ATTTGGCCCA AATTTGGCCT 60
   GCCGCGTCAC CCAAGGCATC GCATCAGTGT AATTCTCTTC GCAATCTGAT TTTTGCTCTG 120
   CTACCCTTCA TGAAAAAAGT CATAACTTCT TGTAGGAAAT ATTGGAATGA TAAATGGTTT 180
   GATGTTCTGG AAACTAGACT CACAGAAAAT TCATTTGATA TATAGCTAAT AGCTCAATTC 240
   GTAATGCATT CGGAGATATG ATTGTTTGAA GTTACATCAT ATGCGAGTAT GCTCGCTTTC 300
   TTCTCTTAAA CCTTTTCTAT TTGTTCCAAA CTACTTCTTC TCACTTATAG ATGTCCATAT 360
   AACTTTACAA ACATGAGATT TAGGTATTAC ACACCTTCAA AACTTTTCGA ACACACGTGT 420
   GTCTACTTAG GGCTTGAACC AGAACGTAAT ACTTAACGAT TTTCGGGGCA TTACATGCAT 480
   ACACCACTGT TAACAGGAAA ATTGCTTTCA TTAAATTATA ACATTGGATT TGGTGTGCAC 540
   TAAGTTCCTA TGCTTAATTG TTATGAACAT GAGTACTTTG CTTTCTCCCT TTGGTGGTGC 600
   ATACTTGTTT GTGGATATAT ATCGAGAATA ATAATGTGAG TGAATAGATA TTGTCTATTA 660
   TTTAACTTTA ATTTGCACCG CTACTTGTTC ACCACATTGG GATTCAATTG GGTGACTCGG 720
   CATATTTATC AATTAATATT CATCTAATGA GAACTCTTGC AAATTCTGTT ATAGGTTCTT 780
   AGTAGCATCA GCTGCATATC ATGTAAACTA AGAGTCAATA TGCTCACTTG TCAGTAAAAA 840
   AGAGTCATTA TCCTCACTTA TGTCATTTAC TCTATAGCTA TATTGGAGGC ATTATGTTAA 900
   TGGATTCCTA ATAATACCAA ATTACACCTT ATATGAGTCA TTGTTGGACA GAGTTTATCA 960
   ATACCTATAT ATTAGTGTAC TCTTATTCTT GCTCTTTGTG AGTATTAATA TGATGACTAT 1020
   ATTGACAGCA TTTGCATGAT GATGAGTGGG GCAGGAGACG CACAAAGTTT GTACCATAGA 1080
   GGAAGTTCGA GTTCTGTGAT AATCTTGGAA GAAAGTATAG TTATATTCTT TCTCCCCACC 1140
   TTGTTGATTT CCGACTTGTT TGAAGTTTGC TCCTTGTTGC TGTCACAATT GTATTCATGT 1200
   TAAGTTCTTT ATGAAGTTGG GTTGACGTTC AAATCTCATA CGCATGTTTG TTGCCTCTTT 1260
   TTATTTGTCT ATGGGGGTTG CATCAGTTGT CTCAGATCAA GATGGGAGCA TATTACTGCT 1320
   CCAAAGGTTT GGTTGTCCTT GGTAGTAACT AGTTCATGTG CAGGTTGGCT GCTCTGTTTG 1380
   ATTCTGCTTT GAGAACTTAA AGCTTTCATT TACTCAATTA TCAAATATCT GGGGTTTAAT 1440
   GGGCTCAAAT CACCCTTATA CAAACACCTT TTGTTTCCCT TATCAATGAA TGAACGAATT 1500
   TCCTTTGAGT TGTGAATGTA ATAAGGGTGT GAAAGAGGAG TTTTCGTTGT TAAATTGGCG 1560
   TTTGAAAGGT TCTCCCTTTT GTTCTTTTTT CGGCTTTTAC TTTTATATAC TGATAGTCTA 1620
   AGAAACTTTT TACACTATCA AGTTGCCTAA AAGATAGCTA CATGAGTAAC TTGTTACAAC 1680
   CGGTTAAATT ACACTAATAT TACAAATAAA AGTAAATCAG TAATATAAAA GTTATTTACA 1740
   TAGTCAATAT ATATAATTTA AATCCTTTTC TATTTTTTCT CGAGGGGTTT GGATTTTTAT 1800
   TTTAGTTGGC TCTTAAGACT TGTGCATGTA CATTCTTGAG AAAATAACTC TGTTCATGAG 1860
   AAAGCTACCT TAACTAACTA ACGTACTTCA CGGCCGAAAC AAAATCATAC AAATAACACA 1920
   TTTCTTTGTG GTTACCTTAA AATTTGGCCA TGAAACTTGG TCTGTTCGAT TATATCTTTA 1980
   AATACTACTA CCATCTACCA CACACTCTCC TCTGTCAAGA TAACAATAAA AGAATAAAAA 2040
   GATTAACCAA AAACGATATA CATATTTAGG ACAGA ATG AAG GTT AGC TTG AAG   2093
                                          Met Lys Val Ser Leu Lys
                                            1               5
   CAC CAC TGG GTA GTG AAG CCA GCA GAG GCA ACA TGG AAT GGC ACT GTC   2141
   His His Trp Val Val Lys Pro Ala Glu Ala Thr Trp Asn Gly Thr Val
                10                  15                  20
   TCC TTA TCG GAG TGT GAT CAA ACT TTT GCT GTA ACT CAT GTA CCA ACC   2189
   Ser Leu Ser Glu Cys Asp Gln Thr Phe Ala Val Thr His Val Pro Thr
            25                  30                  35
   ATT TAT TAC TAC AGG TTT TGC CAT GAT TGT CTT CCA TCA ACA GAC AAT   2237
   Ile Tyr Tyr Tyr Arg Phe Cys His Asp Cys Leu Pro Ser Thr Asp Asn
        40                  45                  50
   ATC ATC AAA ACC CTC AGG ACC TCA CTA AGC AAA GCA TTA GTA CAC TTC   2285
   Ile Ile Lys Thr Leu Arg Thr Ser Leu Ser Lys Ala Leu Val His Phe
    55                  60                  65                  70
   TAT CCA TTG TCT GGT CGT TTG CGA TGG ATC GCT GGG TCC CGC CTC GAG   2333
   Tyr Pro Leu Ser Gly Arg Leu Arg Trp Ile Ala Gly Ser Arg Leu Glu
                    75                  80                  85
   CTC GAC TGT AAT GCC TCG GGA ATC GTG CTC ATG GAA GCT GAA ACC GAA   2381
   Leu Asp Cys Asn Ala Ser Gly Ile Val Leu Met Glu Ala Glu Thr Glu
                90                  95                 100
   GCC AAA CTA GAT GAT CTT GGC GAT TTC TCG CCA TCC CCT GAC TTG AAC   2429
   Ala Lys Leu Asp Asp Leu Gly Asp Phe Ser Pro Ser Pro Asp Leu Asn
           105                 110                 115
   AGC TTG TTT CCC CGT GTA GAC TAC ACA ATC CCA ATT GAT GAA CTC CCT   2477
   Ser Leu Phe Pro Arg Val Asp Tyr Thr Ile Pro Ile Asp Glu Leu Pro
       120                 125                 130
   TTG TTG TTT GTT CAG CTT ACT AAG TTT CAG TGT GGT GGT ATT GCT CTG   2525
   Leu Leu Phe Val Gln Leu Thr Lys Phe Gln Cys Gly Gly Ile Ala Leu
   135                 140                 145                 150
   AGT TTT GCA ATA TCA CAT GCT GTA GTT GAT GGC CAA AGT GCT CTT TAC   2573
   Ser Phe Ala Ile Ser His Ala Val Val Asp Gly Gln Ser Ala Leu Tyr
                   155                 160                 165
   TTC CTC ACC GAA TGG GCT AGC CTT GCT CGC GGA GAG CCA TTA GGG AAC   2621
   Phe Leu Thr Glu Trp Ala Ser Leu Ala Arg Gly Glu Pro Leu Gly Asn
               170                 175                 180
   GAA CCT TTT CAT GAT CGA AAA TTC CTC CGA GCA GGG GAA CCT CCA ATT   2669
   Glu Pro Phe His Asp Arg Lys Phe Leu Arg Ala Gly Glu Pro Pro Ile
   185 190 195
   GCA TAT CCA ACG TTT GAG CAT TTA CAG TTT AAT CCA CCA CCA CTT TTG 2717
   Ala Tyr Pro Thr Phe Glu His Leu Gln Phe Asn Pro Pro Pro Leu Leu
   200 205 210
   CTT GGA CAG TCC AGC AGT GAA GAG GAG AAG AAA AAT GAA ACA AAG GGT 2765
   Leu Gly Gln Ser Ser Ser Glu Glu Glu Lys Lys Asn Glu Thr Lys Gly
   215 220 225 230
   TCC ATG CTA AAA CTT ACA AAA CAT CAA GTT GAA ATG TTG AGA AAA AAG 2813
   Ser Met Leu Lys Leu Thr Lys His Gln Val Glu Met Leu Arg Lys Lys
   235 240 245
   GCG AAC CAA GGT AAT CAA GGG CGT AGT TAC ACA CGT TAT GAA GTT GTG 2861
   Ala Asn Gln Gly Asn Gln Gly Arg Ser Tyr Thr Arg Tyr Glu Val Val
   250 255 260
   ACT GCA CAT ATA TGG AGA TGT GCA TGC AAG GCA AGA GGT CAT AAA TTT 2909
   Thr Ala His Ile Trp Arg Cys Ala Cys Lys Ala Arg Gly His Lys Phe
   265 270 275
   GAG CAG CCT ACT AAT TTA TGC ATT TGT GTT AAC ATA CGC AAT ATA ATG 2957
   Glu Gln Pro Thr Asn Leu Cys Ile Cys Val Asn Ile Arg Asn Ile Met
   280 285 290
   CAA CCA CCT TTG CCT AAA TCC TAT TTT GGC AAT GCC ATA GTT GAT GTT 3005
   Gln Pro Pro Leu Pro Lys Ser Tyr Phe Gly Asn Ala Ile Val Asp Val
   295 300 305 310
   ATT GCC AAT GGC GTC TCG GGT GAC ATT ACC TCG AGG CCA TTG GAG TAT 3053
   Ile Ala Asn Gly Val Ser Gly Asp Ile Thr Ser Arg Pro Leu Glu Tyr
   315 320 325
   GTT GCT CGA AGG GTG CGA GCA GCC ATT AAA ATG GTG ACG AGT GAT TAC 3101
   Val Ala Arg Arg Val Arg Ala Ala Ile Lys Met Val Thr Ser Asp Tyr
               330                 335                 340
   GCA AAC TCG ACG ATT GAT TTC TTA AAA AAC CAG GAG GAT TTG TCA AAA   3149
   Ala Asn Ser Thr Ile Asp Phe Leu Lys Asn Gln Glu Asp Leu Ser Lys
           345                 350                 355
   TAT CAA GAT ATT CAT GCA TTT AGA AGC AAG GAA GGT CCT TTT TAT GGA   3197
   Tyr Gln Asp Ile His Ala Phe Arg Ser Lys Glu Gly Pro Phe Tyr Gly
       360                 365                 370
   AAC CCT AAT CTT GGG GTT ATA AGT TGG ATA AGT TTG CCA TTA TTA GGA   3245
   Asn Pro Asn Leu Gly Val Ile Ser Trp Ile Ser Leu Pro Leu Leu Gly
   375                 380                 385                 390
   TTG GAT TTT GGG TGG GGA AAA GAG ATA CAT ATG AGC CCT GGA ACT CAT   3293
   Leu Asp Phe Gly Trp Gly Lys Glu Ile His Met Ser Pro Gly Thr His
                   395                 400                 405
   GAA TAT GAT GGT GAT TGT GTG ATA CTT CCA GGA AAA GAA GGG GAT GGA   3341
   Glu Tyr Asp Gly Asp Cys Val Ile Leu Pro Gly Lys Glu Gly Asp Gly
               410                 415                 420
   TCT TTG ACT GTT GCA ATC ATT CTT CAA GCT GTT CAT GTG GAT GCT TTC   3389
   Ser Leu Thr Val Ala Ile Ile Leu Gln Ala Val His Val Asp Ala Phe
           425                 430                 435
   AAG AAC TTC TTC TAT GAA GAA ATT GAA TGT TGAAAAACAT AAGTGTTTTA     3439
   Lys Asn Phe Phe Tyr Glu Glu Ile Glu Cys
       440                 445
   TGAGAAGAAA GGAAACAAAT TAAGAACATG TAGCTTTTCC TAAATTGACA TTGTTAGTCA 3499
   TGGTCTAAGC AAAATAAACT CTTTATCTAC ACATTATTTC AATATATTTT CCTTATTTTC 3559
   TATCAGATTT CTCATATGTT TATTTGATGT TCTTAATTTT ACGAACAATA ATCGGTCATA 3619
   AATGGTTTGA AAATCAATAA CCAAAACTGG AACTATATTG ATTGTTTGGA AGCTAAGCAC 3679
   TTTTTTTCTT CTTTTTTCGC AAAGCAC 3706
(2) INFORMATION FOR SEQ ID NO:17:
   (i) SEQUENCE CHARACTERISTICS:
      (A) LENGTH: 448 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Lys Val Ser Leu Lys His His Trp Val Val Lys Pro Ala Glu Ala
    1               5                  10                  15
   Thr Trp Asn Gly Thr Val Ser Leu Ser Glu Cys Asp Gln Thr Phe Ala
               20                  25                  30
   Val Thr His Val Pro Thr Ile Tyr Tyr Tyr Arg Phe Cys His Asp Cys
           35                  40                  45
   Leu Pro Ser Thr Asp Asn Ile Ile Lys Thr Leu Arg Thr Ser Leu Ser
       50                  55                  60
   Lys Ala Leu Val His Phe Tyr Pro Leu Ser Gly Arg Leu Arg Trp Ile
   65                  70                  75                  80
   Ala Gly Ser Arg Leu Glu Leu Asp Cys Asn Ala Ser Gly Ile Val Leu
                   85                  90                  95
   Met Glu Ala Glu Thr Glu Ala Lys Leu Asp Asp Leu Gly Asp Phe Ser
   100 105 110
   Pro Ser Pro Asp Leu Asn Ser Leu Phe Pro Arg Val Asp Tyr Thr Ile
   115 120 125
   Pro Ile Asp Glu Leu Pro Leu Leu Phe Val Gln Leu Thr Lys Phe Gln
   130 135 140
   Cys Gly Gly Ile Ala Leu Ser Phe Ala Ile Ser His Ala Val Val Asp
   145 150 155 160
   Gly Gln Ser Ala Leu Tyr Phe Leu Thr Glu Trp Ala Ser Leu Ala Arg
   165 170 175
   Gly Glu Pro Leu Gly Asn Glu Pro Phe His Asp Arg Lys Phe Leu Arg
   180 185 190
   Ala Gly Glu Pro Pro Ile Ala Tyr Pro Thr Phe Glu His Leu Gln Phe
   195 200 205
   Asn Pro Pro Pro Leu Leu Leu Gly Gln Ser Ser Ser Glu Glu Glu Lys
   210 215 220
   Lys Asn Glu Thr Lys Gly Ser Met Leu Lys Leu Thr Lys His Gln Val
   225 230 235 240
   Glu Met Leu Arg Lys Lys Ala Asn Gln Gly Asn Gln Gly Arg Ser Tyr
   245 250 255
   Thr Arg Tyr Glu Val Val Thr Ala His Ile Trp Arg Cys Ala Cys Lys
   260 265 270
   Ala Arg Gly His Lys Phe Glu Gln Pro Thr Asn Leu Cys Ile Cys Val
   275 280 285
   Asn Ile Arg Asn Ile Met Gln Pro Pro Leu Pro Lys Ser Tyr Phe Gly
   290 295 300
   Asn Ala Ile Val Asp Val Ile Ala Asn Gly Val Ser Gly Asp Ile Thr
   305 310 315 320
   Ser Arg Pro Leu Glu Tyr Val Ala Arg Arg Val Arg Ala Ala Ile Lys
   325 330 335
   Met Val Thr Ser Asp Tyr Ala Asn Ser Thr Ile Asp Phe Leu Lys Asn
   340 345 350
   Gln Glu Asp Leu Ser Lys Tyr Gln Asp Ile His Ala Phe Arg Ser Lys
   355 360 365
   Glu Gly Pro Phe Tyr Gly Asn Pro Asn Leu Gly Val Ile Ser Trp Ile
   370 375 380
   Ser Leu Pro Leu Leu Gly Leu Asp Phe Gly Trp Gly Lys Glu Ile His
   385 390 395 400
   Met Ser Pro Gly Thr His Glu Tyr Asp Gly Asp Cys Val Ile Leu Pro
   405 410 415
   Gly Lys Glu Gly Asp Gly Ser Leu Thr Val Ala Ile Ile Leu Gln Ala
   420 425 430
   Val His Val Asp Ala Phe Lys Asn Phe Phe Tyr Glu Glu Ile Glu Cys
   435 440 445
(2) INFORMATION FOR SEQ ID NO:18:
   (i) SEQUENCE CHARACTERISTICS:
      (A) LENGTH: 1906 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant43D
   (vii) IMMEDIATE SOURCE:
      (B) CLONE: pCIB952
   (ix) OFEATURE:
      (A) NAME/KEY: CDS
      (B) LOCATION: join (1230..1570, 1669..1684)
   (ix) FEATURE:
      (A) NAME/KEY: intron
      (B) LOCATION: 1571..1668
   (ix) FEATURE:
      (A) NAME/KEY: misc_feature
      (B) LOCATION: 1167
      (D) OTHER INFORMATION: /note= "Putative transcriptional start site"
   (xi) SEQUENCE DESCRIPTION: SEQ ID NO:18:
   GAATTCCTCG ATTTAACCAG AAATCTGCAA AAAATCCCTC AATTCAGCTA ATTAGGACTC 60
   TGATACCATG TTAACTTTCA CTGATTTATA CTGATTATGA AGTGTAATCC ACAACTGAAT 120
   GAATTGAGAA GACGAAATTG AAAGCAGAAG AAAGGTAAAG AACAGAGAGA ACAATATGAT 180
   TACTTCTCTG CTTAGCAATG TCGGTCATTA CTAACAAAAT GAATGTATAC ATATACTTAT 240
   ACTAATATTT ATTGACTCCT AATAGATGAC CGTTGTAAAT AAGAAAAATG ACATAATTAC 300
   TCCTGTAGCT AACTAATGAT CAGGGAATTA TAGTGCAATT AACTAACTCC TTTACAAAAC 360
   CCGATTTACT TTGATGCGAT TGACTTTTTC ATATATCTTA ATTTAATGGA AAGAATCTGT 420
   GATTATCACA CCTTATTTAG AGAAGATCTT TTAAAAGTAA GGAGGCATCG CCTAAAACAT 480
   CTTAATAACT TCCTTTTCAC CGCATAAAAT AAGTGTGTAA ACCGTAGTAG TGTGTAAACC 540
   AGCAAAAGAA CAACCATATA AAGAAAAATA TGTGAAATTA TATTTAAGCC GCTCCCAAAA 600
   ATAATAGCCG ATAAAATGTA TATTTTTCAT ACATTATGTG TATGTATTAT ATACGAAAAA 660
   GATACATATT TTATATACTT TTTGACAAAT GAATACAATT AGTTTCGGTC AACCTGCCAA 720
   TTTTATATTT TGCCCTAAAA ATATACCCAA CAAAAAGAGA CTTTGTATGT AAAAAAAAAA 780
   AAAAAATTAC TATGTGCAAA GTTAAGATCG GCAGGCTGCC TTAAAATCCC AAAAAAAAAA 840
   AAAAAAAAAA AAATGGCTTG CTTTAATTAC ACATGAACAG CCAATGGTTT GCTTTAATTT 900
   ATTCCTCTAA TACGTATATT GTCGTTGACA GAGAATTTGA ATCAAGCAAC TCACATCTCC 960
   AAATAGAAGA GGAAATATCG TGTGAAATTC CAATTGAACA ACAAACTGCG CAGAGAATTG 1020
   AAAACTCTAA TTCATGAGAA TCGCATGTTA CAAGTTACTA TAACAGAATA AAGGGGCTGA 1080
   AAGATAGGTA TATATATATA TATATATATA TATATATATA TATATATATA TATATATATA 1140
   TATATATGTC ACTCATTTGC ACATAATTCT ACACACAGAG AGAATTTAAC TTACATTTCT 1200
   TCAATAGTTT AGTCCATAAA GCAATAGAT ATG GCT CGG TTT CTT GTG TTC CTT   1253
                                   Met Ala Arg Phe Leu Val Phe Leu
                                    1              5
   GCT TTA GCC CTT GTA ATA ATT TCA AAG AAG GGC GCG TTG GGT GCT CCT   1301
   Ala Leu Ala Leu Val Ile Ile Ser Lys Lys Gly Ala Leu Gly Ala Pro
        10                  15                  20
   CCT TCC TGT CCA ACA GTT ACA ACG CAG CTG GCT CCT TGT CTA TCG TAC   1349
   Pro Ser Cys Pro Thr Val Thr Thr Gln Leu Ala Pro Cys Leu Ser Tyr
    25                  30                  35                  40
   ATT CAA GGT GGA GGT GAT CCA TCT GTA CCT TGC TGC ACT GGT ATA AAT   1397
   Ile Gln Gly Gly Gly Asp Pro Ser Val Pro Cys Cys Thr Gly Ile Asn
                    45                  50                  55
   AAC ATA TAT GAA CTT GCT AAA ACC AAA GAA GAC CGA GTC GCT ATC TGC   1445
   Asn Ile Tyr Glu Leu Ala Lys Thr Lys Glu Asp Arg Val Ala Ile Cys
                60                  65                  70
   AAC TGC TTA AAA ACC GCA TTT ACT CAT GCT GGA AAT GTC AAT CCC ACT   1493
   Asn Cys Leu Lys Thr Ala Phe Thr His Ala Gly Asn Val Asn Pro Thr
            75                  80                  85
   CTC GTA GCT CAA CTC CCC AAG AAA TGT GGC ATT TCT TTT AAT ATG CCT   1541
   Leu Val Ala Gln Leu Pro Lys Lys Cys Gly Ile Ser Phe Asn Met Pro
        90                  95                 100
   CCT ATT GAT AAA AAC TAC GAC TGT AAC  AC  GTAAGTTTAT ATTACCTCTC    1590
   Pro Ile Asp Lys Asn Tyr Asp Cys Asn  Thr
   105                 110
   AATTTTTATT TCCACCCAAT TTGGTGCAGA TCGACTGCTT GTTTAATCTA ACTTATTATT 1650
   TTTATTACAT GCATGCAG G ATT TCT ATG TAC TGATGAATGG GTAGTGAATC       1701
   Ile Ser Met Tyr
   115
   TCGGAAGCTG CTCAAATTTA TGAATAAAAC ATATATAGAT GTTCATCTCA TGTCTGAAAT 1761
   CTGAAAGCAA TTTGATCCAC TGTAAACTTC AAATGTATGC AGACGGTTAA ATGTTGAATT 1821
   ATGATATATA TAAATTTGGT TAATGCCTTT GTTTTTGGTA GTCTTAGACC AAGTTCACCA 1881
   AGAGAGACGG TTCATATGAG CTTTT 1906
(2) INFORMATION FOR SEQ ID NO:19:
   (i) SEQUENCE CHARACTERISTICS:
      (A) LENGTH: 118 amino acids
      (B) TYPE: amino acid
      (D) TOPOLOGY: linear
   (ii) MOLECULE TYPE: protein
   (xi) SEQUENCE DESCRIPTION: SEQ ID NO:19:
   Met Ala Arg Phe Leu Val Phe Leu Ala Leu Ala Leu Val Ile Ile Ser
   1 5 10 15
   Lys Lys Gly Ala Leu Gly Ala Pro Pro Ser Cys Pro Thr Val Thr Thr
   20 25 30
   Gln Leu Ala Pro Cys Leu Ser Tyr Ile Gln Gly Gly Gly Asp Pro Ser
   35 40 45
   Val Pro Cys Cys Thr Gly Ile Asn Asn Ile Tyr Glu Leu Ala Lys Thr
   50 55 60
   Lys Glu Asp Arg Val Ala Ile Cys Asn Cys Leu Lys Thr Ala Phe Thr
   65 70 75 80
   His Ala Gly Asn Val Asn Pro Thr Leu Val Ala Gln Leu Pro Lys Lys
   85 90 95
   Cys Gly Ile Ser Phe Asn Met Pro Pro Ile Asp Lys Asn Tyr Asp Cys
   100 105 110
   Asn Thr Ile Ser Met Tyr
   115
(2) INFORMATION FOR SEQ ID NO:20:
   (i) SEQUENCE CHARACTERISTICS:
      (A) LENGTH: 437 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Nicotiana tabacum
      (C) INDIVIDUAL ISOLATE: Ant43C
   (ix) FEATURE:
      (A) NAME/KEY: CDS
      (B) LOCATION: 167..436
   (xi) SEQUENCE DESCRIPTION: SEQ ID NO:20:
   CTGTGATTAA GGATACTGTC ACCCCTGTGA ATTTGGTTGG ATATGGGTTG GCTTTCTTGG   60
   GTGACAGTAT CCTTAATCAC AGACCAAGAA AAGGCAATCA ACAACCAATC CTACACACAC  120
   ACATTTAAAT TACATTTCTT CAATTGTAGT CCATAAACCA ATAGAT ATG GCT CGG     175
                                                      Met Ala Arg
                                                        1
   TTT CTT GCT TTA GCC CTA GTA GTT ATA GCT CTC TCA AAC GAC GCG TTG    223
   Phe Leu Ala Leu Ala Leu Val Val Ile Ala Leu Ser Asn Asp Ala Leu
         5                  10                  15
   GGT GCT CCT CCC TCG TGT CAA ACT GTT ACA ACG CAG CTG GCT CCT TGT    271
   Gly Ala Pro Pro Ser Cys Gln Thr Val Thr Thr Gln Leu Ala Pro Cys
    20                  25                  30                  35
   CTA TCG TAC ATT CAA AAT CGT GTT AAG GGC GGT GGC AAT CCA TCA GTA    319
   Leu Ser Tyr Ile Gln Asn Arg Val Lys Gly Gly Gly Asn Pro Ser Val
                    40                  45                  50
   CCT TGT TGT ACC GGT ATA AAT AAC ATA TAT GAA CTC GCT AAA ACC AAA    367
   Pro Cys Cys Thr Gly Ile Asn Asn Ile Tyr Glu Leu Ala Lys Thr Lys
                55                  60                  65
   GAA GAT CGA GTC GCT ATC TGC AAC TGC TTA AAA AAC GCA TTT ATT CAT    415
   Glu Asp Arg Val Ala Ile Cys Asn Cys Leu Lys Asn Ala Phe Ile His
            70                  75                  80
   GCT GGA AAT GTC AAT CCC ACC C                                      437
   Ala Gly Asn Val Asn Pro Thr
        85                  90
(2) INFORMATION FOR SEQ ID NO:21:
   (i) SEQUENCE CHARACTERISTICS:
      (A) LENGTH: 90 amino acids
      (B) TYPE: amino acid
      (D) TOPOLOGY: linear
   (ii) MOLECULE TYPE: protein
   (xi) SEQUENCE DESCRIPTION: SEQ ID NO:21:
   Met Ala Arg Phe Leu Ala Leu Ala Leu Val Val Ile Ala Leu Ser Asn
   1 5 10 15
   Asp Ala Leu Gly Ala Pro Pro Ser Cys Gln Thr Val Thr Thr Gln Leu
   20 25 30
   Ala Pro Cys Leu Ser Tyr Ile Gln Asn Arg Val Lys Gly Gly Gly Asn
   35 40 45
   Pro Ser Val Pro Cys Cys Thr Gly Ile Asn Asn Ile Tyr Glu Leu Ala
   50 55 60
   Lys Thr Lys Glu Asp Arg Val Ala Ile Cys Asn Cys Leu Lys Asn Ala
   65 70 75 80
   Phe Ile His Ala Gly Asn Val Asn Pro Thr
   85 90

## Description

The present invention is in the field of plant genetic engineering. It primarily relates to novel DNA sequences derived from the Ant43D genomic clone which function as promoters of anther-specific transcription of associated expressible and preferably coding DNA sequences in recombinant or chimeric DNA sequences. The present invention also relates to recombinant or chimeric DNA sequences containing the promotes of the invention which are expressed specifically in the anther of a plant. The said recombinant or chimeric DNA sequences may be used to create transgenic plants, but especially transgenic male-sterile plants. The invention further describes anther-specific cDNA sequences and genomic DNA sequences which may be suitably used in the isolation of the anther-specific promoter sequences according to the invention.

The creation of male sterile plants is of economic interest in the production of hybrid seeds. Male sterility prevents self-pollination which otherwise occurs in many plant species and hinders breeding and hybrid seed production.

Transcription of many plant genes is controlled in a temporal and spatial manner. Regulation of gene activity is mediated by the interaction of trans acting factors and cis regulatory elements in the promoter region of a gene.

Of particular interest are genes which are expressed primarily or exclusively in the sexual tissue of the plant, such as anther or pollen tissue. Such genes can be used to express polypeptides that are not naturally produced in the anther or pollen. For example, the promoter region from an anther specific gene may be used to express a polypeptide which will disrupt formation of viable pollen when expressed in the anther cells, resulting in a male sterile plant.

European Patent Application 0 420 819 A1 describes the use of wun1 gene to produce male sterile plants.

United States Patent 5,086,169 describes the isolation of the promoter region from the Zm13 clone of a pollen-specific gene of corn, and its use to express genes in pollen.

PCT WO 89/10396 describes the use of male-sterility DNAs and anther-specific cDNAs TA13, TA26 and TA29. The developmental expression profiles of TA13 and TA29 matched two cDNA clones isolated by the applicants, ANTS and ANT45, respectively.

PCT WO 90/08825 describes three gene sequences, pMS10, pMS 14 and pMS18, and their use in recombinant DNA sequences with the GUS reporter gene. No evidence of expression is given.

PCT WO 90/08831 describes a disrupter gene known as the mammalian uncoupling protein (UCP) gene which inhibits respiration in cells.

PCT WO 90/08828 describes molecular methods of hybrid seed production in which recombinant DNA molecules using pollen specific promoters are used to control the production of fertile pollen in plants.

It is therefore the main object of the present invention to provide DNA sequences derived from the Ant43D genomic clone which are capable of directing the anther-specific expression of genes in plants. It is a further object of the invention to provide recombinant or chimeric DNA sequences containing the promotes according to the invention which are expressed specifically in the anther of a plant and vectors comprising the said recombinant or chimeric DNA sequences. The said DNA sequences or vectors may be used to create transgenic plants, but especially transgenic male-sterile plants.

The anther-specific genomic clone Ant43D, may be suitably used for the preparation of anther-specific promoter DNA sequences and ligating the said promoter sequences in an operable manner to expressible DNA sequences that encode a desired expression product.

Thus, according to the present invention, novel DNA sequences according to the invention are provided which are expressed specifically in the anther of a plant.

In one embodiment, the present invention comprises an isolated nucleotide sequence consisting essentially of an anther-specific cDNA sequence. The cDNA sequence may be obtained by differential screening of cDNA libraries and selecting those cDNA clones which are observed to be expressed in a highly specific manner in anther tissue. The cDNA clones of the present invention may have the DNA sequences of SEQ. ID No. 3,

In another embodiment, the present invention comprises an isolated genomic DNA sequence, designated Ant43D, which essentially consists of a DNA sequence that corresponds to an anther-specific cDNA clone and is thus capable of hybridizing with the said anther-specific cDNA. The genomic DNA sequence of the present invention may thus be preferably obtained by hybridization of a genomic library with the anther-specific cDNA sequence used as a probe. The genomic DNA sequence of the present invention may have the DNA sequences of SEQ. ID No. 18, or may be obtained by hybridization with a cDNA having the DNA sequence of SEQ. ID No. 3.

The genomic DNA clone is being preferably used as a source for the isolation of DNA sequences which function as promoters of anther-specific transcription of associated expressible and preferably coding DNA sequences in recombinant or chimeric DNA constructs.

It goes without saying that the DNA sequences according to the invention, once having been identified, do not have to be newly isolated each time from a suitable source but can of course be synthesised very easily at any time by means of known chemical processes. Suitable processes for the synthesis of short DNA oligonucleotides, for example the phosphotriester or phosphite method, are known to the person skilled in the art. Today, the majority of oligonucleotide syntheses are mechanised and automated, so that short DNA fragments can be produced in a short period of time.

By deletion, insertion or substitution of one or more base pairs in the above-mentioned DNA sequences according to the invention, therefore, variants or mutants of those sequences can very easily be produced and checked for their suitability as an anther-specific promoter sequence.

An especially suitable method of producing specific mutants is so-called oligonucleotide-mediated mutagenesis. In that method, short oligonucleotide fragments are synthesised which, although substantially homologous to the wild-type sequence, differ therefrom in individual nucleotides. The said differences may be insertions, deletions, inversions or a substitution of one or more nucleotides, or they may be a combination of the above-mentioned procedures. These mutated fragments are then substituted for the homologous counterparts on the wild-type gene by generally known methods. The final construct can then, as described above, be cloned into a suitable plant expression vector and transformed into a plant.

However, the mutation of certain DNA fragments can also preferably be carried out using the polymerase chain reaction [PCR]. In this *in vitro* process there are used chemically synthesised oligonucleotides which generally originate from the peripheral regions of the DNA fragment to be mutated and are strand-specific. Under suitable conditions, hybridisation of the oligonucleotides with the complementary regions on the DNA single strands produced by denaturing occurs. The double-stranded regions produced in this manner are used as primers for the subsequent polymerase reaction.
In this process there may be used in addition to DNA polymerases from *E. coli* especially heat-stable polymerases from thermophilic bacteria, for example *Thermus aquaticus*.

In yet another embodiment, the present invention comprises isolated recombinant, or chimeric, DNA sequences in which a promoter region obtainable from the anther-specific genomic DNA sequence according to the invention is operatively linked to an expressible DNA sequence which preferably encodes a protein which is desired to be expressed in the anther cells. For example, the isolated recombinant DNA sequences of the present invention may comprise, in a 5' to 3' direction, a promoter region obtained from anther-specific genomic DNA sequence ID n°3 operatively linked to a DNA sequence which encodes a polypeptide which will disrupt formation of viable pollen when expressed in the anther cells. The resulting plant will not be able to produce viable pollen cells, and hence will be male sterile. Examples of such a recombinant DNA sequence include chimeric vectors in which an anther-specific promoter is operatively linked to a DNA sequence which encodes a polypeptide selected from the group consisting of the coding sequence from the DTA, TURF-13, pectate lyase, gin recombinase, iaaL or cytA toxin genes.

The present invention thus further relates to a process for the production of a recombinant DNA molecule comprising, in a 5' to 3' direction, an anther-specific promoter region according to the invention operatively linked to an expressible DNA sequence, which process comprises
(a) first isolating from a suitable source or synthesising by means of known processes a promoter region responsible for the anther-specific expression of an associated expressible DNA sequence; and
(b) operably linking the said anther-specific DNA sequence in a 5' to 3' direction to an expressible DNA sequence.

In order to specifically direct the location of the peptide encoded by the recombinant DNA sequence within the plant cell, the recombinant DNA sequence of the present invention may comprise, in a 5' to 3' direction, an anther-specific promoter region operably linked to a signal sequence, which is operably linked to a coding DNA sequence.

Another embodiment of the present invention comprises plasmids containing anther-specific promoter sequences of the present invention but especially recombinant, or chimeric, DNA sequences in which a promoter region obtainable from an anther-specific genomic DNA sequence is operatively linked to an expressible DNA sequence which preferably encodes a protein which is desired to be expressed in the anther cells. These plasmids include, but are not limited to pCIB3178. The present invention also includes promoter fragments that may be derived from the plasmids of the present invention, which still exhibit those characteristics that are essential for carrying out the invention, that is, which are capable of driving the expression of an associated DNA sequence in an anther-specific manner.

For the purpose of the present invention, the term "derived from" a plasmid refers to the physical isolation of a nucleotide sequence or fragment from a plasmid, as well as the physical isolation of a nucleotide sequence or fragment using a probe homologous to one of the above plasmids, or a synthetic nucleotide sequence prepared by using some or all of the nucleotide sequences of the above plasmids.

Another embodiment of the present invention comprises transgenic plants and the sexual and/or asexual progeny thereof, which plants have been transformed with a recombinant, or chimeric, DNA sequence comprising an anther-specific promoter sequence according to the invention and, optionally, a signal sequence operatively linked in a 5' to 3' orientation to an expressible DNA sequence, but preferably to a coding DNA sequence. Such transgenic plants will express the polypeptide coded by the chimeric DNA sequence only in the anther of the plant. When the polypeptide encoded is a polypeptide which will disrupt formation of viable pollen when expressed in the anther cells, the transgenic plant will not be able to produce viable pollen cells, and hence will be male sterile. For example, such transgenic plants may encode for DTA, TURF-13, pectate lyase, gin recombinase, iaaL or cytA toxin.

It is thus still a further embodiment of the present invention to provide male-sterile plants transformed with one of the above recombinant, or chimeric, DNA sequences.

The present invention further comprises a process for the production of transformed plant material, including whole plants, comprising a recombinant DNA molecule comprising, in a 5' to 3' direction, an anther-specific promoter region according to the invention operatively linked to an expressible DNA sequence, which process essentially comprises:
(a) first of all isolating from a suitable source or synthesising by means of known processes a promoter region responsible for the anther-specific expression of an associated expressible DNA sequence;
(b) operably linking the said anther-specific DNA sequence in a 5' to 3' direction to an expressible DNA sequence;
(c) cloning the final construct into a plant expression vector under the control of expression signals active in plants;
(d) transforming the said expression vector into plant material by means of known processes and expressing it therein; and optionally
(e) regenerating the plant material transformed according to step (d) to a whole and preferably phaenotypically normal plant.

### Definitions:

"Anther-specific" is used to describe cDNAs, genomic DNAs, messenger RNAs, promoter DNA sequences and genes which are associated with anther tissue. In the case of cDNAs, genomic DNAs and messenger RNAs, "anther-specific" describes the fact that, when assayed through northern blot hybridization, the mRNA corresponding to the cDNA, genomic DNA or mRNA sequence is present in anther tissue in concentrations at least about 100-fold that observed in other tissues. In the case of promoter DNA sequences, "anther-specific" describes a regulatory sequence which directs the transcription of associated coding sequences so that the corresponding messenger RNA is present in anther tissue in concentrations at least about 100-fold that observed in other tissues. In the case of a gene, "anther-specific" describes a gene which is expressed in a manner so that the gene product is present in anther tissue in concentrations at least about 100-fold that observed in other tissues. Because anther and pollen tissue are both involved in the male sexual function of a plant, a DNA sequence or gene may be considered to be "anther-specific" for the purpose of the present invention if it is expressed specifically in pollen as well as in anther tissues.

"Recombinant" and "chimeric" are both used to indicate that a DNA sequence, vector or gene is comprised of more than one DNA sequence of distinct origin which have been fused or ligated together, resulting in a DNA sequence, vector or gene which does not occur naturally. For example, the ligation of a promoter DNA sequence from an anther-specific gene with the coding DNA sequence of a different gene of heterologous origin is said to be "recombinant" or "chimeric".

In the following description, a number of expressions are used that are customary in recombinant DNA technology and in plant genetics. In order to ensure a clear and unifonn understanding of the description and the claims and also of the scope to be accorded to the said expressions, the following definitions are listed.

Plant material: Parts of plants that are viable in culture or that are viable as such, such as protoplasts, cells, callus, tissue, embryos, plant organs, buds, seeds, etc., and also whole plants.

Plant cell: Structural and physiological unit of the plant, comprising a protoplast and a cell wall. The plant cell may be in form of an isolated single cell or as a part of higher organized unit such as, for example, a plant tissue, a plant organ or a whole plant.

Protoplast: "Naked" plant cell that has no cell wall and has been isolated from plant cells or plant tissues and has the potential to regenerate to a cell clone or a whole plant.

Plant tissue: Group of plant cells organised in the form of a structural and functional unit.

Plant organ: Structural and functional unit comprising several tissues, for example root, stem, leaf or embryo.

Heterologous gene(s) or DNA: A DNA sequence that codes for a specific product or products or fulfils a biological function and that originates from a species other than that into which the said gene is to be inserted; the said DNA sequence is also referred to as a foreign gene or foreign DNA.

Homologous gene(s) or DNA: A DNA sequence that codes for a specific product or products or fulfils a biological function and that originates from the same species as that into which the said gene is to be inserted.

Synthetic gene(s) or DNA: A DNA sequence that codes for a specific product or products or fulfils a biological function and that is produced by synthetic means.

The present invention relates to anther-specific nucleotide sequences derived from the genomic clone Ant43D an especially to recombinant, or chimeric, DNA sequences which are expressed in much higher amounts in the anther of a plant than in other tissue. The said recombinant, or chimeric, DNA sequences comprise a promoter region obtainable from said anther-specific genomic DNA sequence, which is operatively linked to an expressible DNA sequence which preferably encodes a protein desired to be expressed in the anther cells.

In a especially preferred embodiment, the coding DNA sequence encodes a polypeptide which, when expressed in the anther cells, will disrupt formation of viable pollen.

Preferred as the coding DNA sequence are sequences which encode a polypeptide selected from the group consisting of DTA, TURF-13, pectate lyase, gin recombinase, iaaL and cytA toxin.

The anther-specific promoter DNA sequences to be used in the recombinant, or chimeric, construct according to the invention are preferably isolated from genomic clones and are ligated in a 5' to 3' orientation to expressible DNA sequences, which preferably encode a desired protein product, to provide chimeric vectors that are specifically expressed in the anther of a plant.

The invention relates to the anther-specific cDNA clone ant43D, corresponding to the sequence of SEQ ID No. 3.

All further cDNA and genomic sequences described below and in the examples are added purely for descriptive purposes.

Thus, further anther-specific genomic DNA sequences corresponding to the anther-specific cDNA clones of SEQ ID Nos. 1, 5, 7, 9, 11, 13, 14, and 20, can be isolated.

Further anther-specific genomic clones may be obtained by using anther-specific cDNA clones as probes to pull out the coresponding genomic DNA clones. Corresponding genomic clones are those which are transcribed to form a messenger RNA which is complementary to and transcribed into a given cDNA. An example of such a genomic clones is the isolated anther-specific genomic DNA clone ant32, the sequence of which is provided at SEQ ID No. 16.

Several cDNA sequences are described which may be used as probes to isolate anther-specific genomic DNA sequences. Examples are the isolated anther-specific cDNA clones ant32, ant9, ant52, ant59, ant66, ant67, ant68 and ant43C, corresponding to the sequences of SEQ ID No. 1, SEQ ID No. 5, SEQ ID No. 7, SEQ ID No. 9, SEQ ID No. 11, SEQ ID No. 13, SEQ ID No. 14 and SEQ ID No. 20, respectively.

The anther-specific cDNA sequences can be obtained by preparing cDNA libraries from anther tissue and leaf tissue. Single stranded DNA from the leaf is photobiotinylated and hybridized to the anther DNA. Photobiotinylated DNA is removed, leaving a library enriched for anther-specific cDNA sequences. (Example 3). Anther-specific cDNAs are identified by differential screening (Example 4)). The anther-specific cDNAs are cross-hybridized to identify unique cDNAs. (Example 4). Anther-specific expression is verified by RNA blot hybridization with various plant tissues and *in situ* hybridization. (Examples 5 and 8). Developmental expression, sequences and gene copy number of the anther-specific cDNA clones is also determined. (Examples 6 and 7 and 9).

The cDNA sequences can be used to isolate genomic DNA sequences. Where a partial cDNA has been obtained, the partial cDNA is used as a probe to screen the anther cDNA library in order to isolate a full length cDNA clone. Hybridizing clones are purified, restriction mapped and sequenced. A full length clone will be near message size as well as having a complete open reading frame. To isolate a genomic clone, the full length anther cDNA is used as a probe to screen a genomic library. By restriction mapping and hybridization to the anther cDNA, the coding region of the genomic clone is identified. The area upstream from the coding area of the clone is the anther promoter region.

The anther-specific promoter region may be more precisely mapped through deletion analysis. 5' deletions of an anther promoter are made by introducing restriction sites by PCR using oligonucleotide primers with restriction sites at the 5' ends and anther promoter sequences at the 3' ends. The PCR products are digested, purified, and cloned into pBI101 (Clontech). The deletion mutants contain the 5' untranslated leader sequence fused to the translational start site of the GUS gene. Internal and 3' deletions of anther promoters are made by PCR in a similar manner. The PCR fragments are fused to a GUS vector containing the CAMV 35S minimal promoter [-46 to +1; Benfey *et al*, 1990]. Transgenic plants are tested with the GUS fluorometric and histochemical assay.

The anter-specific promoter region may be suitably used for the preparation of recombinant, or chimeric, DNA constructs comprising an expressible DNA sequence but preferable a coding DNA sequence, that is specifically expressed in the anther of a plant.

The present invention thus further describes recombinant DNA sequences comprising, in a 5' to 3' direction, a promoter region obtained from an anther-specific genomic DNA sequence, ID n°3 which is operatively linked to a coding DNA sequence. The recombinant DNA sequences result in anther-specific expression of the associated expressible DNA, which preferably is a coding DNA sequence which encodes for a structural gene.

The coding DNA sequence of the present invention may be any DNA sequence encoding for a desired polypeptide. Preferred within the scope of the present invention are, for example, all those structural genes which lead to a protective effect in the transformed plant cells and also in the tissues developing therefrom and especially in the plants, for example increased resistance to pathogens (for example to phytopathogenic fungi, bacteria, viruses, etc.); resistance to chemicals [for example to herbicides (e.g. triazines, sulfonylureas, imidazolinones, triazole pyrimidines, bialaphos, glyphosate, etc.), insecticides or other biocides]; resistance to adverse environmental factors (for example to heat, cold, wind, adverse soil conditions, moisture, dryness, etc.).

Further coding DNA sequences to be used within the scope of the present invention may be those that lead to the production of desirable and useful compounds in the plant cell.

Genes that may also be used within the scope of the present invention include, for example, those which lead to increased formation of reserve or stored substances in leaves, seeds, tubers, roots, stems, etc. or in the protein bodies of seeds. The desirable substances that can be produced by transgenic plants include, for example, proteins, carbohydrates, amino acids, vitamins, alkaloids, flavins, perfumes, colourings, fats, etc..

There may also be associated with the DNA sequence according to the invention structural genes that code for pharmaceutically acceptable active substances, for example hormones, immunomodulators and other physiologically active substances.

Especially preferred for use in the present invention are, however, coding DNA sequences which encode the production of a polypeptide which, when expressed in anther tissue, will result in the inability of the plant to produce viable pollen. Examples of such coding DNA sequences include the genes which are described in the following references, the disclosures of which are hereby incorporated by reference as if fully set forth herein:
a) Diptheria toxin A-chain gene (DTA), which inhibits protein synthesis, Greenfield *et al*, 1983; Palmiter *et al*, 1987.
b) Pectate lyase gene pelE from Erwinia chrysanthemi EC16, which degrades pectin, causing cell lysis. Keen *et al*, 1986.
c) T-urf13 (TURF-13) gene from cms-T maize mitochondrial genomes; this gene encodes a polypeptide designated URF13 which disrupts mitochondrial or plasma membranes. Braun et *al*, 1990; Dewey *et al*, 1987; and Dewey et *al*, 1986.
d) Gin recombinase gene from phage Mu gene, which encodes a site-specific DNA recombinase which will cause genome rearrangements and loss of cell viability when expressed in cells of plants. Maeser *et al*, 1991.
e) Indole acetic acid-lysine synthetase gene (iaaL) from Pseudomonas syringae, which encodes an enzyme that conjugates lysine to indoleacetic acid (IAA). When expressed in the cells of plants, it causes altered development due to the removal of IAA from the cell via conjugation. Romano *et al*, 1991; Spena *et al*, 1991; Roberto *et al*, 1990.
f) CytA toxin gene from *Bacillus thuringiensis Israeliensis* which encodes a protein that is mosquitocidal and hemolytic. When expressed in plant cells, it causes death of the cell due to disruption of the cell membrane. McLean *et al*, 1987; Ellar *et al*, United States Patent No. 4,918,006 (1990).

The recombinant DNA sequences described in the present invention may further comprise, in a 5' to 3' direction, a promoter region obtained from an anther-specific genomic DNA sequence, ID n°3 operatively linked to a signal sequence, which is operatively linked to a coding DNA sequence. The signal sequence is responsible for specialized transport of the associated peptide within the plant cell.

The signal sequence may be any DNA sequence which is able to direct the transport of and associated polypeptide into one or more of the cellular compartments. The signal sequence is preferably a sequence which is translated into a signal peptide, which becomes separated from the peptide after transit of the peptide is complete. Signal sequences are useful for directing the polypeptide product of the coding DNA sequence to a desired location within the cell, such as to the mitochondria or to the endoplasmic reticulum, or to direct extracellular transport outside of the cell. Among the signal sequences useful for the present invention are, for example, the signal sequence from the pathogenesis-related gene (PR-1) of tobacco, which is described in Cornellisen *et al*, 1986; the yeast mitochondrial presequence; Schmitz *et al*, 1989; the signal sequence from plant mitochondrial Rieske iron-sulfur protein, Huang *et al*, 1991; mitochondrial and chloroplast targeting peptides, von Heijne *et al*, 1989. The identification of other leader sequences is known in the art. See Della-Cioppa *et al*, 1987; Schekman, 1985.

The various sections of sequence can be linked to one another by methods known *per se* to form a complete coding DNA sequence. Suitable methods include, for example, the *in vivo* recombination of DNA sequences having homologous sections and the *in vitro* linking of restriction fragments.

As cloning vectors there are generally used plasmid or virus (bacteriophage) vectors having replication and control sequences originating from species that are compatible with the host cell.

The cloning vector generally carries an origin of replication, especially an origin of replication that is capable of functioning in *E. coli*, in *Agrobacterium* or in both, and, in addition, specific genes that lead to phenotypic selection features in the transformed host cell, especially to resistance to antibiotics or to specific herbicides. The transformed vectors can be selected on the basis of those phenotypic markers after transformation in a host cell.

Selectable phenotypic markers that may be used within the scope of this invention include, for example, resistance to ampicillin, tetracycline, hygromycin, kanamycin, methotrexate, G418 and neomycin, but this list, which is given by way of example, is not intended to limit the subject of the invention.

Suitable host cells within the scope of this invention are prokaryotes, including bacterial hosts, for example *A. tumefaciens, E. coli, S. typhimurium* and *Serratia marcescens*, and also cyanobacteria. Eukaryotic hosts, such as yeasts, mycelium-forming fungi and plant cells, may also be used within the scope of this invention.

The splicing of the hybrid gene construction according to the invention into a suitable cloning vector is carried out using standard methods, such as those described, for example, in Maniatis *et al* (1982) or Sambrook *et al* (1989).

The cloning vectors and the host cell transformed with those vectors are generally used to increase the number of copies of the constructs cloned therein. With an increased number of copies it is possible to isolate the vector carrying the hybrid gene construction and prepare it, for example, for insertion of the chimaeric gene sequence into a plant cell.

In a further process step, these plasmids are used to insert the structural genes coding for a desired gene product or non-coding DNA sequences having a regulatory function into a plant cell and, optionally, to integrate them into the plant genome.

The present invention therefore also describes the production of recipient plant cells that comprise the said structural gene or other desirable genes or gene fragments or other useful DNA sequences incorporated in their genome.

A number of very efficient processes have come into existence for introducing DNA into plant cells, which processes are based on the use of gene transfer vectors or on direct gene transfer processes.

One possible method comprises, for example, bringing plant cells into contact with viruses or with *Agrobacterium*. This may be achieved by infecting sensitive plant cells or by co-cultivating protoplasts derived from plant cells. Within the scope of this invention, Cauliflower Mosaic Virus (CaMV) may also be used as a vector for the insertion of the chimaeric genetic construction according to the invention into a plant.

Another method of inserting the chimaeric gene construction according to the invention into a cell makes use of the infection of the plant cell with *Agrobacterium tumefaciens* and/or *Agrobacterium rhizogenes*, which has been transformed with the said gene construction. The transgenic plant cells are then cultured under suitable culture conditions known to the person skilled in the art, so that they form shoots and roots and whole plants are finally formed.

Using newly developed transformation techniques, it has also become possible in principle to transform *in vitro* plant species that are not natural host plants for *Agrobacterium* [Grimsley NH *et al* (1987)]. For example, monocotyledonous plants, especially the cereal species and various grasses, are not natural hosts for *Agrobacterium*.

Preferred within the scope of this invention is so-called leaf disk transformation using *Agrobacterium* [Horsch *et al* (1985)]. Sterile leaf disks from a suitable target plant are incubated with *Agrobacterium* cells comprising one of the chimaeric gene constructions according to the invention, and are then transferred into or onto a suitable nutrient medium. Especially suitable, and therefore preferred within the scope of this invention, are LS media that have been solidified by the addition of agar and enriched with one or more of the plant growth regulators customarily used, especially those selected from the group of the auxins consisting of a-naphthylacetic acid, picloram, 2,4,5-trichlorophenoxyacetic acid, 2,4-dichlorophenoxyacetic acid, indole-3-butyric acid, indole-3-lactic acid, indole-3-succinic acid, indole-3-acetic acid and p-chlorophenoxyacetic acid, and from the group of the cytokinins consisting of kinetin, 6-benzyladenine, 2-isopentenyladenine and zeatin. The preferred concentration of auxins and cytokinins is in the range of from 0.1 mg/l to 10 mg/l.

After incubation for several days, but preferably after incubation for 2 to 3 days at a temperature of from 20°C to 40°C, preferably from 23°C to 35°C and more especially at 25°C and in diffuse light, the leaf disks are transferred to a suitable medium for the purpose of shoot induction. Especially preferred for the selection of the transformants is an LS medium that does not contain auxin but contains cytokinin instead, and to which a selective substance has been added. The cultures are kept in the light and are transferred to fresh medium at suitable intervals, but preferably at intervals of one week. Developing green shoots are cut out and cultured further in a medium that induces the shoots to form roots. Especially preferred within the scope of this invention is an LS medium that does not contain auxin or cytokinin but to which a selective substance has been added for the selection of the transformants.

In addition to *Agrobacterium*-mediated transformation, within the scope of this invention it is possible to use direct transformation methods for the insertion of the gene constructions according to the invention into plant material.

For example, the genetic material contained in a vector can be inserted directly into a plant cell, for example using purely physical procedures, for example by microinjection using finely drawn micropipettes [Neuhaus *et al* (1987)] or by bombarding the cells with microprojectiles that are coated with the transforming DNA ["Microprojectile Bombardment"; Wang Y-C *et al* (1988)].

Other possible methods for the direct transfer of genetic material into a plant cell comprise the treatment of protoplasts using procedures that modify the plasma membrane, for example polyethylene glycol treatment, heat shock treatment or electroporation, or a combination of those procedures [Shillito *et al* (1985)].

A further method for the direct introduction of genetic material into plant cells, which is based on purely chemical procedures and which enables the transformation to be carried out very efficiently and rapidly, is described in Negrutiu I *et al* (1987) and in Goodall G *et al*.

Also suitable for the transformation of plant material is direct gene transfer using co-transformation (Schocher RJ *et al* 1986).

The list of possible transformation methods given above by way of example does not claim to be complete and is not intended to limit the subject of the invention in any way.

The present invention therefore also comprises transgenic plant material, selected from the group consisting of protoplasts, cells, calli, tissues, organs, seeds, embryos, ovules, zygotes, etc. and, especially, whole and preferably male-sterile plants, that has been transformed by means of the processes described above and comprises the recombinant DNA according to the invention in expressible form, and processes for the production of the said transgenic plant material.

The process for the production of transformed plant material, including whole plants, comprising an expression product that is expressed in an anther-specific manner essentially comprises:
(a) first of all isolating from a suitable source or synthesising by means of known processes a DNA sequence responsible for the anther-specific expression of an associated expressible DNA sequence;
(b) operably linking the said anther-specific DNA sequence in a 5' to 3' direction to an expressible DNA sequence;
(c) cloning the final construct into a plant expression vector under the control of expression signals active in plants;
(d) transforming the said expression vector into plant material by means of known processes and expressing it therein; and optionally
(e) regenerating the plant material transformed according to step (d) to a whole and preferably phaenotypically normal plant.

The present invention thus also comprises transgenic plants and the sexual and/or asexual progeny thereof, which have been transformed with a recombinant DNA sequence according to the invention comprising the promoter region from an anther-specific genomic DNA sequence.

The expression "asexual or sexual progeny of transgenic plants" includes by definition according to the invention all mutants and variants obtainable by means of known processes, such as for example cell fusion or mutant selection and which still exhibit the characteristic properties of the initial transformed plant, together with all crossing and fusion products of the transformed plant material.

Another object of the invention concerns the proliferation of material of transgenic plants according to the invention. The proliferation material of transgenic plants is defined relative to the invention as any plant material that may be propagated sexually or asexually *in vivo* or *in vitro.* Particularly preferred within the scope of the present invention are protoplasts, cells, calli, tissues, organs, seeds, embryos, egg cells, zygotes, together with any other propagating material obtained from transgenic plants.

### Description of the Sequences

Sequence 1 is the nucleotide sequence of anther-specific cDNA clone ant32.

Sequence 2 is the amino acid sequence of the polypeptide encoded by the ant32 nucleotide sequence of Sequence 1.

Sequence 3 is the nucleotide sequence of anther-specific cDNA clone ant43D.

Sequence 4 is the amino acid sequence of the polypeptide encoded by the ant43D nucleotide sequence of Sequence 3.

Sequence 5 is the nucleotide sequence of anther-specific cDNA clone ant9.

Sequence 6 is the amino acid sequence of the polypeptide encoded by the ant9 nucleotide sequence of Sequence 5.

Sequence 7 is the nucleotide sequence of anther-specific cDNA clone ant52.

Sequence 8 is the amino acid sequence of the polypeptide encoded by the ant52 nucleotide sequence of Sequence 7.

Sequence 9 is the nucleotide sequence of anther-specific cDNA clone ant59.

Sequence 10 is the amino acid sequence of the polypeptide encoded by the ant59 nucleotide sequence of Sequence 9.

Sequence 11 is the nucleotide sequence of anther-specific cDNA clone ant66.

Sequence 12 is the amino acid sequence of the polypeptide encoded by the ant66 nucleotide sequence of Sequence 11.

Sequence 13 is the nucleotide sequence of anther-specific cDNA clone ant67.

Sequence 14 is the nucleotide sequence of anther-specific cDNA clone ant68.

Sequence 15 is the amino acid sequence of the polypeptide encoded by the ant68 nucleotide sequence of Sequence 14.

Sequence 16 is the nucleotide sequence of the Ant32 genomic clone. This sequence shows the nucleotide sequence of the ant32 gene, including 2.0 kb of 5' flanking sequence. The TATA box is found at bases 1971 to 1975. The putative transcription start site is found at base 2009. Bases 2009 to 2075 comprise the untranslated leader sequence. The ATG translational initiation codon is found at bases 2076 to 2078. No introns are present. The TGA stop codon is found at bases 3420 to 3422.

Sequence 17 is the amino acid sequence of the polypeptide encoded by the Ant32 nucleotide sequence of Sequence 16.

Sequence 18 is the nucleotide sequence of the Ant43D genomic clone. This sequence shows the nucleotide sequence of the ant43D gene, including approximately 1.2 kb of 5' flanking sequence. The putative transcriptional start site is found at base 1167. An unusually long TATA box is found at bases 1089 to 1147. The sequence "TA" is repeated 29 times. The untranslated leader is found between bases 1167 and 1229. The translational initiation codon occurs at bases 1230 to 1232. Translated sequences are shown in uppercase. One intron occurs at bases 1571 to 1668.

Sequence 19 is the amino acid sequence of the polypeptide encoded by the Ant43D nucleotide sequence of Sequence 18.

Sequence 20 is the nucleotide sequence of anther-specific cDNA clone ant43C.

Sequence 21 is the amino acid sequence of the polypeptide encoded by the ant43C nucleotide sequence of Sequence 20.

### Description of the Figures

**Figure 1: Restriction map of Ant32 genomic clone pCIB950.** The arrow indicates the location of the ant32 gene, as well as its 5' to 3' orientation in genomic subclone pCIB950. The promoter region extends from the upstream PstI site to the coding region.

**Figure 2: Restriction map of Ant43D genomic clone pCIB952.** The arrow indicates the location of the ant43D gene, as well as its 5' to 3' orientation in genomic subclone pCIB952. The promoter region extends from the upstream EcoRI site to the coding region.

**Figure 3:** Site-specific mutagenesis via PCR resulting in insertion of a XbaI site before the start of translation of Ant32 (Figure 3A) and Ant43D (Figure 3B).

In Figure 3A, the drawing on the top left shows the 3' end of the PstI-SacI ant32 genomic subclone containing the promoter. Underneath it is the sequence at the ATG before which an XbaI site was inserted as described in Example 16.

In Figure 3B, the drawing on the top left shows the 3' end of the EcoRI ant43D genomic subclone containing the promoter. Underneath it is the sequence at the ATG before which an XbaI site was inserted as described in Example 18.

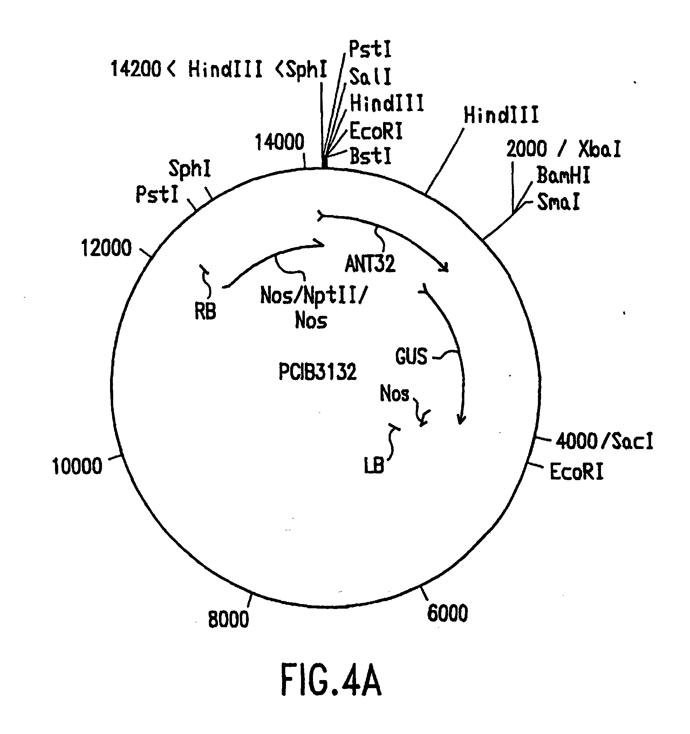

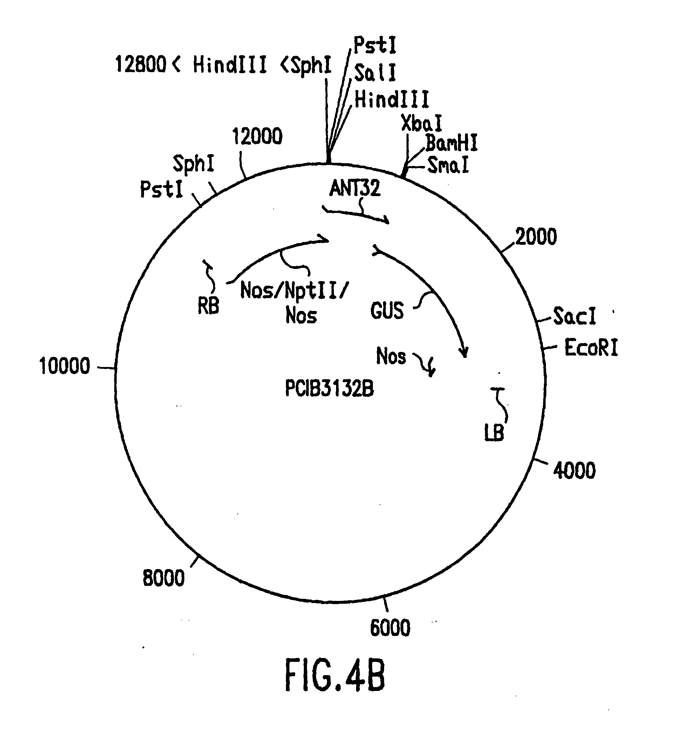

**Figure 4: Plasmid maps of Ant32-GUS fusions** pCIB3132 (2.0 kb promoter - Figure 4A) and pCIB3132B (600 bp promoter - Figure 4B), pCIB3132 has been deposited with the USDA Agricultural Research Service Culture Collection, Northern Regional Research Center (NRRL) at 1815 North University Street, Peoria, Ill. 61604, on June 16, 1992 and has been accorded deposit no. NRRL B-18977.

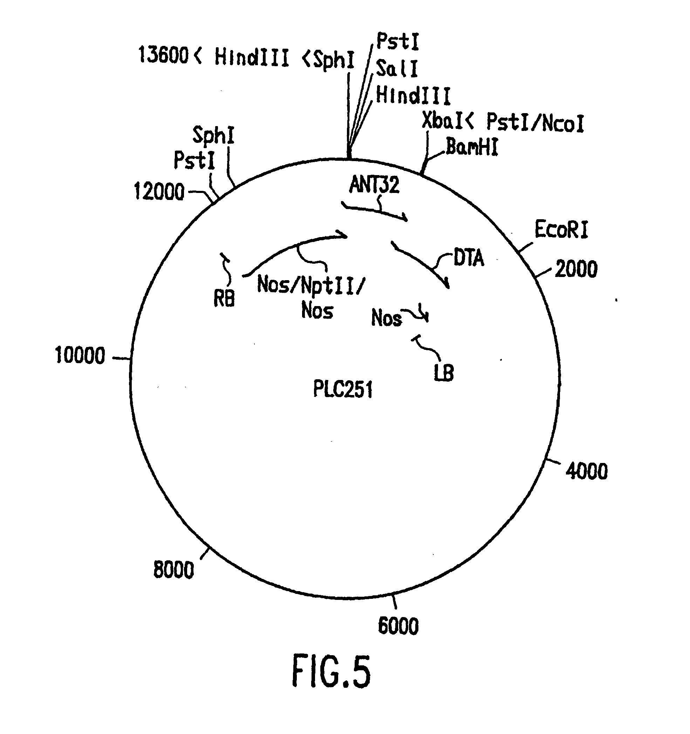

### Figure 5: Plasmid map of Ant32-DTA fusion pLC251

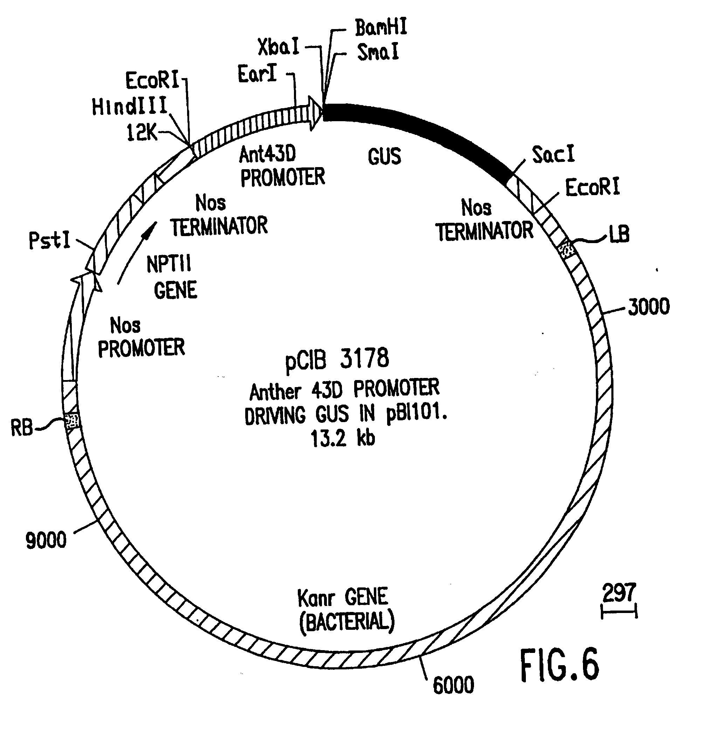

**Figure 6: Plasmid map of Ant43D-GUS fusion pCIB3178.** pCIB3178 has been deposited with the USDA NRRL on June 16, 1992 and has been accorded deposit no. NRRL B-18978.

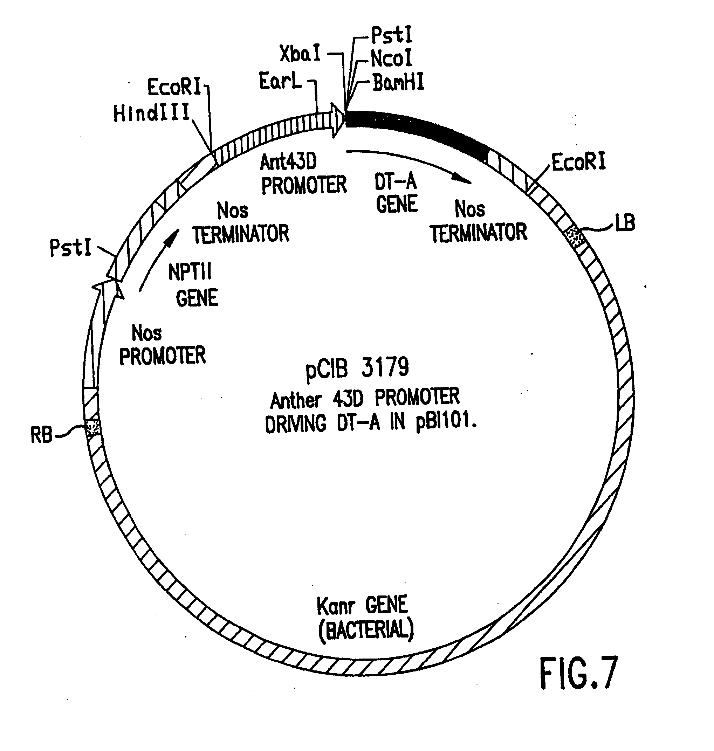

### Figure 7: Plasmid map of Ant43D-DTA fusion pCIB3179.

The following examples further describe the materials and methods used in carrying out the invention. They are offered by way of illustration, and their recitation should not be considered as a limitation of the claimed invention.

### NON-LIMITING EXAMPLES

### General recombinant DNA techniques

Since many of the recombinant DNA techniques employed in this invention are a matter of routine for the person skilled in the art, it is better to give a short description of these generally used techniques here rather than to describe them every time they occur. Except where there is a specific indication to the contrary, all these procedures are described in the Maniatis *et al* (1982) reference.

### A. Cleaving with restriction endonucleases

A reaction batch typically contains about 50 to 500 µg/ml of DNA in the buffer solution recommended by the manufacturer, New England Biolabs, Beverly, MA.. 2 to 5 units of restriction endonucleases are added for each µg of DNA and the reaction batch is incubated for from one to three hours at the temperature recommended by the manufacturer. The reaction is terminated by heating at 65°C for 10 minutes or by extraction with phenol, followed by precipitation of the DNA with ethanol. This technique is also described on pages 104 to 106 of the Maniatis *et al* (1982) reference.

### B. Treatment of DNA with polymerase in order to produce blunt ends

50 to 500 µg/ml of DNA fragments are added to a reaction batch in the buffer recommended by the manufacturer, New England Biolabs. The reaction batch contains all four deoxynucleotide triphosphates in concentrations of 0.2 mM. The reaction takes place over a period of 30 minutes at 15°C and is then terminated by heating at 65°C for 10 minutes. For fragments obtained by cleaving with restriction endonucleases that produce 5'-projecting ends, such as EcoRI and BamHI, the large fragment, or Klenow fragment, of DNA polymerase is used. For fragments obtained by means of endonucleases that produce 3'-projecting ends, such as PstI and SacI, the T4 DNA polymerase is used. The use of these two enzymes is described on pages 113 to 121 of the Maniatis *et al* (1982) reference.

### C. Agarose gel electrophoresis and purification of DNA fragments from gels

Agarose gel electrophoresis is carried out in a horizontal apparatus, as described on pages 150 to 163 of the Maniatis *et al* reference. The buffer used is the tris-borate buffer described therein. The DNA fragments are stained using 0.5 µg/ml of ethidium bromide which is either present in the gel or tank buffer during electrophoresis or is added after electrophoresis. The DNA is made visible by illumination with long-wave ultraviolet light. If the fragments are to be separated from the gel, an agarose is used that gels at low temperature and is obtainable from Sigma Chemical, St. Louis, Missouri. After the electrophoresis, the desired fragment is cut out, placed in a plastics test tube, heated at 65°C for about 15 minutes, extracted three times with phenol and precipitated twice with ethanol. This procedure is slightly different from that described by Maniatis *et al* (1982) on page 170.

As an alternative, the DNA can be isolated from the agarose with the aid of the Geneclean kit (Bio 101 Inc., La Jolla, CA, USA).

### D. Addition of synthetic linker fragments to DNA ends

If it is desired to add a new endonuclease cleavage site to the end of a DNA molecule, the molecule is optionally first treated with DNA-polymerase in order to produce blunt ends, as described in the section above. About 0.1 to 1.0 µg of this fragment is added to about 10 ng of phosphorylated linker DNA, obtained from New England Biolabs, in a volume of 20 to 30 µl with 2 µl of T4 DNA ligase from New England Biolabs, and 1 mM ATP in the buffer recommended by the manufacturer. After incubation overnight at 15°C, the reaction is terminated by heating at 65°C for 10 minutes.

The reaction batch is diluted to about 100 µl in a buffer appropriate for the restriction endonuclease that cleaves the synthetic linker sequence. About 50 to 200 units of this endonuclease are added. The mixture is incubated for 2 to 6 hours at the appropriate temperature, then the fragment is subjected to agarose gel electrophoresis and purified as described above. The resulting fragment will then have ends with endings that were produced by cleaving with the restriction endonuclease. These ends are usually cohesive, so that the resulting fragment can then readily be linked to other fragments having the same cohesive ends.

### E. Removal of 5'-terminal phosphates from DNA fragments

During the plasmid cloning steps, treatment of the vector plasmid with phosphatase reduces the recircularisation of the vector (discussed on page 13 of the Maniatis *et al* reference). After cleavage of the DNA with the correct restriction endonuclease, one unit of calf intestinal alkaline phosphatase obtained from Boehringer-Mannheim, Mannheim, is added. The DNA is incubated at 37°C for one hour and then extracted twice with phenol and precipitated with ethanol.

### F. Linking of DNA fragments

If fragments having complementary cohesive ends are to be linked to one another, about 100 ng of each fragment are incubated in a reaction mixture of 20 to 40 µl containing about 0.2 unit of T4 DNA ligase from New England Biolabs in the buffer recommended by the manufacturer. Incubation is carried out for 1 to 20 hours at 15°C. If DNA fragments having blunt ends are to be linked, they are incubated as above except that the amount of T4 DNA ligase is increased to 2 to 4 units.

### G. Transformation of DNA into E. coli

*E. coli* strain HB101 is used for most of the experiments. DNA is introduced into *E. coli* using the calcium chloride method, as described by Maniatis *et al* (1982), pages 250 and 251.

### H. Screening of E. coli for plasmids

After transformation, the resulting colonies of *E. coli* are tested for the presence of the desired plasmid by means of a rapid plasmid isolation process. Two customary processes are described on pages 366 to 369 of the Maniatis *et al* (1982) reference.

### I. Large-scale isolation of plasmid DNA

Processes for the isolation of plasmids from *E. coli* on a large scale are described on pages 88 to 94 of the Maniatis *et al* (1982) reference.

### J. Cloning in M13 phage vectors

In the following description it is to be understood that the double-stranded replicative form of the phage M13 derivatives is used for routine processes, such as cleaving with restriction endonuclease, linking etc..

Unless there is a specific indication to the contrary, enzymes can be obtained from Boehringer, Biolabs (BRL). They are used in accordance with the manufacturer's instructions unless otherwise indicated.

### K. Southern blot analysis

The extracted DNA is first treated with restriction enzymes, then subjected to electrophoresis in a 0.8 % to 1 % agarose gel, transferred to a nitrocellulose membrane [Southern EM (1975)] and hybridised with the DNA to be detected which has previously been subjected to nick-translation (DNA-specific activities of 5 x 10⁸ to 10 x 10⁸ c.p.m./µg). The filters are washed three times for 1 hour each time with an aqueous solution of 0.03M sodium citrate and 0.3M sodium chloride at 65°C. The hybridised DNA is made visible by blackening an X-ray film over a period of 24 to 48 hours.

The examples given below and which do not relate to the cDNA clone ant43D, corresponding to seq. ID n°3, are added purely for descriptive purposes and not part of the invention.

### Examples

### Example 1: Plant Material and Growth Conditions

Tobacco plants (Nicotiana tabacum cv Xanthi) are grown from seed in a greenhouse under a 16-hour light/ 8-hour dark light regime.

### Example 2: Anther and Leaf mRNA Isolation

Total RNA is isolated from anthers from 0 to 10mm pistil length flower buds and from 5 week old seedlings by the Phenol/SDS method described by Ausubel *et al*, (1987). PolyA+ RNA is purified from total RNA as described by Maniatis *et al*, (1982).

### Example 3: Construction of Subtracted cDNA Libraries

Anther and seedling cDNA libraries are made using INVITROGEN's Libraviam II kit [INVITROGEN CORP, 3985B Sorrento Valley Blvd, San Diego, CA; Cat No L1958-15]. Double-stranded cDNA is synthesized from anther and leaf polyA+ RNA, BstXI non-palindromic linkers are ligated on and the cDNA is cloned into a BstXI cut pTZ18R-B vector, which is available from INVITROGENE CORP. [3985B Sorrento Valley Blvd., San Diego, CA]. Transformation is into E. coli DH1aF' cells, which can also be purchased from INVITROGENE CORP. A subtraction cDNA library is made using INVITROGEN's Subtractor kit [INVITROGEN CORP, 3985B Sorrento Valley Blvd, San Diego, CA; Cat No K4320-01]. Single-stranded DNA is isolated from the anther and leaf cDNA libraries. The leaf single-stranded DNA is photobiotinylated and hybridized to the anther single-stranded DNA. Both hybridized and unhybridized photobiotinylated sequences are removed with streptavidin and phenol extraction. The remaining DNA is converted to double-stranded form with Klenow and transformed into E. coli DH1aF' cells.

### Example 4: Isolation of Anther-Specific cDNA Clones

Anther-specific clones are identified by differential screening of the anther subtraction cDNA library. 20,000 clones are replica plated onto nitrocellulose filters and differentially screened to identify colonies hybridizing to radioactively labeled first strand cDNA from anther polyA+ RNA but not to first strand cDNA from seedling polyA+. The cDNAs inserted into the cloning vector are cut out with a restriction enzyme and the inserts of althogether 70 cDNAs are differentially screened again by Southern blot. Northern blots of anther, pistil and leaf total RNA are probed with the cDNAs to confirm tissue specificity. All anther-specific cDNAs are cross-hybridized to identify unique cDNAs. Unique cDNA clones are purified and subcloned into bluescript vector. A full-length cDNA clone of ant32 is isolated by screening the anther cDNA library with a .9 kb partial cDNA. The two cDNAs are 95% homologous at the sequence level, and are therefore closely related members of the same gene family.

### Example 5: Verification of Expression Pattern by RNA Blot Hybridization

Northern blots are done using nitrocellulose filters as described in Maniatis (1982). 20µg of anther, pistil and leaf total RNA are loaded per lane. Prehybridizations are done at 68°C for 4 hours in 3X SSC, 5X Denhardt's, 20mM Tris pH 7, .1% SDS, 2mM EDTA and 1005g/ml sheared denatured salmon sperm DNA. Hybridizations are done at 42°C overnight in 6X SSC, 5X Denhardt's, .1% SDS, 500 µg/ml salmon sperm DNA, 8% dextron sulfate and 50% formamide to which 5.5 x 10⁶ cpm/ml of probe is added. Probes are synthesized using PHARMACIA's oligolabelling kit [PHARMACIA LKB BIOTECHNOLOGY, 800 Centennial Avenue, Piscataway, NJ; Cat No 27-9250-01] in accordance with the manufacturer's instructions. Expression of the cDNAs is seen only in anther RNA. Expression in pollen is also seen with the ant66 cDNA.

PolyA+ RNA is isolated from anther, pistil, leaf, petal, stem and root tissue. 15g of each along with 205g of seed and sepal total RNA are run on a Northern and probed with the ant32 and ant43D cDNAs. Expression of both cDNAs is seen only in anther RNA, demonstrating that the ant32 and ant43D cDNAs are tightly regulated and expressed only in anther tissue.

### Example 6: Developmental Expression of Anther-Specific cDNA Clones

Total RNA is isolated from anthers from 6 stages of flower bud lengths. Slot blots are probed with the anther-specific cDNAs to determine developmental expression. Slot hybridization is done as in Maniatis (1982) using 105g of RNA. Table 1 contains the developmental expression profile of the cDNAs. Ant9, 32, 43C, 59, and 68 are expressed only early in anther development, whereas ant43D, 52 and 67 are expressed throughout development. Ant66 is expressed only late in development.

### Example 7: Sequencing of Anther-Specific cDNA Clones

DNA is sequenced using the dideoxy chain-termination method of Sanger *et al*, 1977, using double-stranded plasmid DNA as a template. All DNA sequence analysis is carried out on a Digital Vax 8530 computer using the University of Wisconsin Computer Genetics Group software, which is commerically available from GENETICS COMPUTER GROUP, INC [University Research Park 575 Schience Drive, Madison, WI]. The oligonucleotide primers are synthesized on an Applied Biosystems Model 380A Synthesizer.

Table 1 contains a comparison of message size to insert size of the anther-specific cDNAs. The ant32 and ant43D cDNAs are close to the size expected for full length copies of the mRNAs. The rest of the cDNAs are incomplete clones. Ant32, 43C, 43D, 52, 59, 66 and 68 encode a single open reading frame. The ant32 cDNA is a near full-length clone of 1542 bases (SEQUENCE ID NO. 1). The sequence contains a large open reading frame which extends from nucleotide 66 to 1412, encoding a complete polypeptide of 448 amino acids. The open reading frame is flanked by 5' and 3' non-coding regions of 65 and 130 bases respectively. A polyadenylation signal, AATAAA, occurs at position 1502.

The ant43D cDNA is a near full-length clone of 552 bases (SEQUENCE ID NO. 3). The sequence contains a complete open reading frame of 118 amino acids, extending from bases 41 to 397. The open reading frame is flanked by 40 bases on the 5' end and 155 bases on the 3' end. A polyadenylation signal is found starting at position 437.

Ant43C is an incomplete cDNA of 437 bases (SEQUENCE ID NO. 20). A partial polypeptide of 90 amino acids is encoded by nucleotides 167 to 436. The ant43C cDNA and the ant43D cDNA are 90% homologous at the sequence level.

Ant52, an incomplete cDNA clone of 96 bases (SEQUENCE ID NO. 7) contains an open reading frame of 31 amino acids.

Ant59 is an incomplete cDNA clone of 1201 bases (SEQUENCE ID NO. 9). An open reading frame extending from nucleotide 1 to 1119 encodes a partial polypeptide of 372 amino acids. The open reading frame is flanked by a 3' non-coding region of 82 bases.

Ant66 is an incomplete cDNA clone of 952 bases (SEQUENCE ID NO. 11). A partial polypeptide of 236 amino acids is encoded by nucleotides 1 to 711. The open reading frame is flanked by a 3' region of 241 bases. The sequence contains a polyA tail of 15 bases.

Ant68 is an incomplete cDNA clone of 445 bases (SEQUENCE ID NO. 20. An open reading frame of 148 amino acids is encoded by the sequence.

Ant67 is an incomplete cDNA clone of 305 bases (SEQUENCE ID NO. 13). It is unknown which strand is the sense strand since a single large open reading frame was not found. This clone contains the 3' end of an open reading frame and a 3' flanking region in translations of both strands.

Ant9 is an incomplete, chimeric cDNA of 612 bases (SEQUENCE ID NO. 5). Northerns of anther, pistil, and leaf tissue are probed with 5' and 3' regions of the chimeric cDNA to determine the anther-specific region of the cDNA clone. Northerns probed with bases 1 to 325 hybridize to anther, pistil and leaf tissue. This region of the cDNA encodes an open reading frame. Northerns probed with bases 326 to 612 hybridize exclusively to anther tissue. This region is identified as the anther-specific region of the chimeric cDNA. A partial polypeptide of 32 amino acids is encoded by nucleotides 344 to 442. A polyadenylation signal starts at position 461.

Each deduced amino acid sequence is compared to sequences in GenBank Genetic Sequence Data Bank, a computer data base of DNA sequences. The ant66 cDNA had a 74% overall amino acid identity with a plasma membrane proton ATPase (H+-ATPase) from Arabadopsis thaliana. Harpear *et al*, 1989. The ant68 cDNA encodes a glycine-rich protein.

### Example 8: Verification of Ant32 Expression Pattern by In-Situ Hybridization

In situ hybridization studies with paraffin-embedded anther sections from 12mm long flower buds are carried out as described by Perez-Grau *et al*, 1989. ³⁵S-RNA probes used for *in situ* hybridizations are synthesized using STRATAGENE's RNA Transcription Kit [STRATAGENE CLONING SYSTEMS, 11099 North Torrey Pines Rd, La Jolla, CA; Cat No 200340]. Cross sections and longitudinal sections are probed with ant32 antisense and sense RNA probes. Expression is localized in the tapetal cell layer of the anther with the antisense probe.

### Example 9: Gene Copy Number

In order to determine how many genes in the tobacco genome hybridize with the anther-specific genes, Xanthi genomic DNA was digested with XbaI, HindIII, EcoRI, and BamHI. Southern blots are probed with the cDNA clones. The blots probed with ant32, 43, 52, 59 and 67 had 2 bands hybridizing in each digest, indicating that these cDNAs are single copy genes or members of small gene families. More bands per digest hybridized in the blots probed with ant9, 66 and 68, indicating that these cDNAs are members of larger gene families.

### Example 10: Southern Blots

Southern blots are done with nitrocellulose as described in Maniatis (1982). Prehybridizations are in 6X SSC, 10X Denhardt's, .2% SDS, and 75 µg/ml salmon sperm DNA at 68°C for 4 to 6 hours. Hybridizations are done at 68°C in 6X SSC, 5X Denhardt's, .5% SDS and 125 µg/ml salmon sperm DNA to which 1 x 10⁶ cpm/ml DNA probe is added. Washes are as described in Maniatis (1982). Genomic Southern blots are done with Duralon-UV membranes (Stratagene) and hybridization conditions are as in the manufacturer's directions.

### Example 11: Construction of Tobacco Genomic DNA Libraries

Tobacco DNA is isolated from leaves using the method of Shure *et al*, (1983). Sau3AI partial digests of Xanthi genomic DNA are cloned into the BamHI site of Stratagene's Lambda DashII vector and the library is amplified. Another genomic library is made using PROMEGA's LambdaGEM-11 XhoI Half-Site Arms Cloning System [PROMEGA, 2800 Woods Hollow Road, Madison, WI; Cat No B1960]. Partially filled-in Sau3AI digested genomic DNA is cloned into partially filled-in XhoI LambdaGEM-11 arms.

### Example 12: Isolation and Sequencing of the Ant32 Genomic Clone

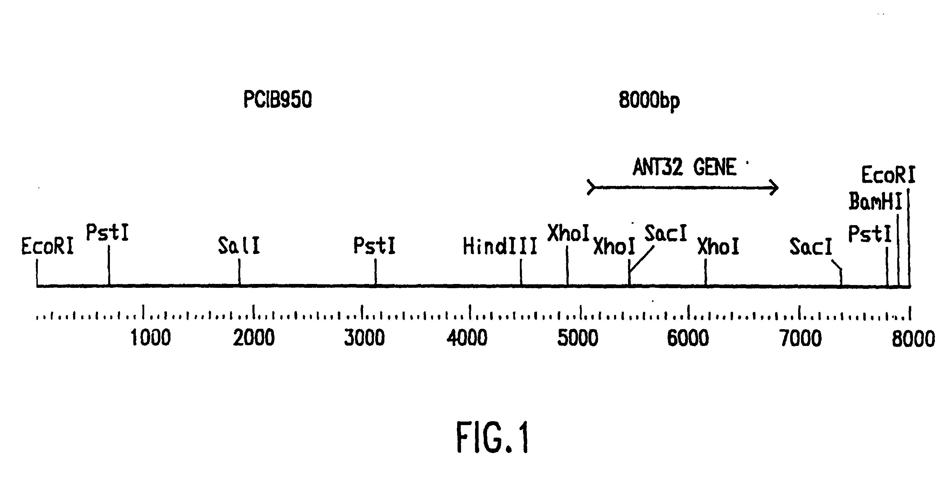

The amplified Stratagene genomic library is screened with ant32 as a probe, yielding 4 hybridized placques. All four clones are purified and restriction mapped. When probed with ant32, one EcoRI fragment from each clone hybridized. The EcoRI fragments are subcloned in plasmid pBS SK⁺, which is available from STRATAGENE CLONING SYSTEMS [11099 North Torrey Pines Road, La Jolla, CA]. Subcloning and mapping of the EcoRI fragments from the 4 clones showed that 2 are identical. Figure 1 contains the map of EcoRI subclone pCIB950. Fragments from pCIB950 are then subcloned for sequencing. 2.0 kb of promoter, the entire coding region, and .28 kb of 3' untranslated region is sequenced. The 2.0 kb promoter fragment from ant32 is functional. As shown in Example 16, a .6kb fragment from ant32 is sufficient to confer anther specific activity.

### Example 13: Isolation and Sequencing of the Ant43D Genomic Clone

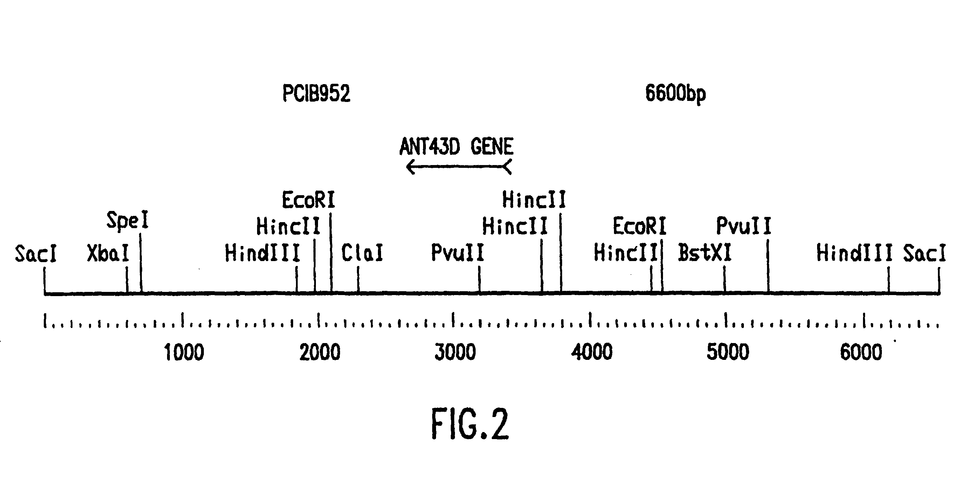

The LambdaGEM-11 primary library is screened with ant43D as a probe. Hybridizing placques are rescreened by PCR to distinguish between ant43D and ant43C, a closely related cDNA. PCR fragments generated from the placques are digested to distinguish between genomics correlating to the 2 cDNAs. Two genomic clones correspond to ant43D, and they are purified and mapped. A 6.6 kb SacI band from both hybridizes to an ant43D probe. Subcloning and mapping of both SacI bands shows that they are identical. Figure 2 contains the map of the SacI subclone, pCIB952. Fragments from pCIB952 are subcloned and sequenced. 1.2 kb of promoter, the entire coding region including one intron, and .22 kb of 3' untranslated region is sequenced. The 1.2 kb promoter fragment contains the entire ant43D promoter.

### Example 14: Primer Extension

The primer is end-labeled using [λ-³²P] ATP (6000 Ci/mmole, Amersham) and T4 polynucleotide kinase. 20µg of anther total RNA is mixed with .01 pmole of primer in 20µl of reverse transcriptase buffer (50mM Tris pH8.3, 75mM KCl, 3mM MgCl₂). The mixture is heated at 80°C for 10 min., annealed by slowly cooling to 40°C, and hybridized overnight at 40°C. To each 20µl reaction is added 30µl of 5mM DTT, .1mg/ml BSA, 1mM each of dATP, dCTP, dGTP, and dTTP in reverse transcriptase buffer containing 200 units of RNAsin (Promega) and 400 units of MMLV reverse transcriptase (BRL). Primer extension is carried out at 40°C for 60 min. The DNA/RNA hybrid is extracted once with phenol:chloroform and ethanol precipitated in the presence of carrier DNA. The pellet is dissolved in sequencing loading dye and analyzed on a 6% acrylamide-urea sequencing gel.

The primers used for the primer extension experiments are as follows:

### Example 15: Mapping the Transcript Start Site by Primer Extension

The start of transcription of the ant32 cDNA and the ant43D cDNA are mapped using primer extension. The largest primer extension product falls within a few base pairs of the end of the ant32 cDNA. The largest primer extension product falls 23 base pairs upstream of the end of the ant43D cDNA.

### Example 16: Fusions of the ant32 promoter sequence to the GUS gene

The 2.0 kb 5' flanking region of pCIB950 containing the ant32 promoter is fused to the bacterial reporter gene for glucuronidase (GUS) in order to characterize the promoter of the anther-specific gene in transgenic plants. An XbaI site is inserted before the ATG by PCR as follows:

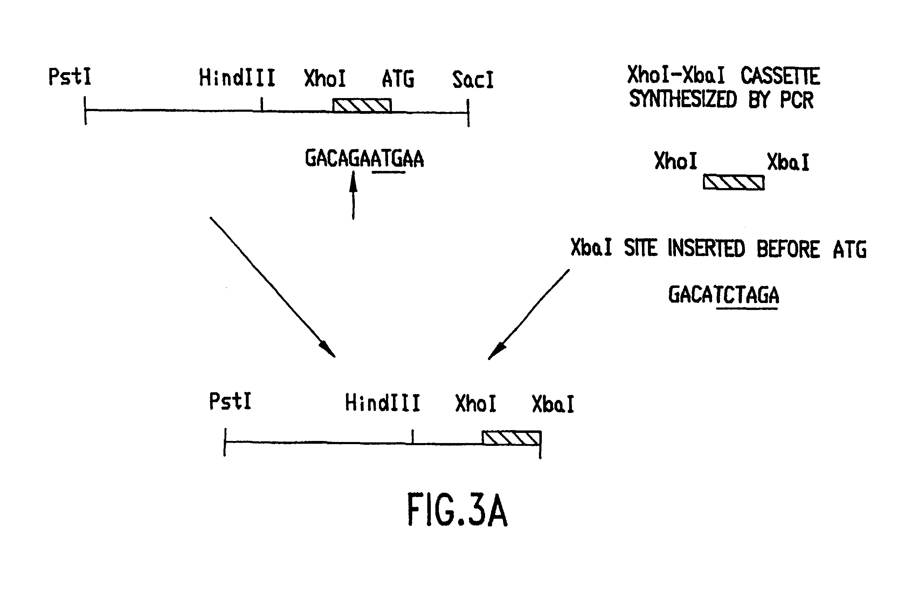

A 350 bp XhoI-XbaI fragment (top right of figure 3A) is synthesized using polymerase chain reaction (PCR) technology [see Mullis *et al*, 1987; Erlich 1989] to copy the ant32 promoter sequence from 55 bp before the unique XhoI site to two bp before the ATG. One of the PCR primers inserts an XbaI site 2bp before the ATG. A full-length ant32 promoter consisting of the PstI-XhoI fragment from the original clone and the XhoI-XbaI PCR cassette is reassembled in a 3-way ligation into the PstI-XbaI sites of the Bluescript vector pBluescript SK (Stratagene). This promoter clone can be used for transcriptonal fusions to coding sequences.

The resulting promoter is excised as a SalI - XbaI fragment and fused to the GUS gene in pBI101 (Clontech). A 600 base pair ant32 promoter - GUS fusion is constructed by deleting a 1.4 kb HindIII fragment from the bluescript promoter clone. The deleted promoter is excised as a SalI - XbaI fragment and fused to the GUS gene in pBI101. The 2.0 kb promoter-GUS fusion is designated pCIB3132 and the 0.6 kb promoter-GUS fusion is designated pCIB3132B. The 0.6 kb promoter fragment from ant32 is sufficient to confer anther specific activity.

### Example 17: Fusion of the ant32 promoter sequence to the DTA gene

A chimeric gene is constructed using a 5' ant32 promoter sequence and the Diptheria toxin A-chain (DTA) coding sequence [Palmiter *et al*, 1987]. The GUS coding sequence is excised from pCIB3132B with SmaI and SacI, the SacI site is filled in, and the plasmid is religated back together (pLC250). The DTA coding sequence is ligated as a BglII fragment, cut out of plasmid p72 [Palmiter *et al*, 1987], into the BamHI site of pLC250, resulting in pLC251. The DTA coding sequence is fused in the opposite orientation in pLC252.

### Example 18: Fusion of the ant43D promoter sequence to the GUS gene

The 1.2 kb 5' flanking region of the ant43D gene is fused to GUS. An XbaI site is inserted before the ATG by PCR as follows:

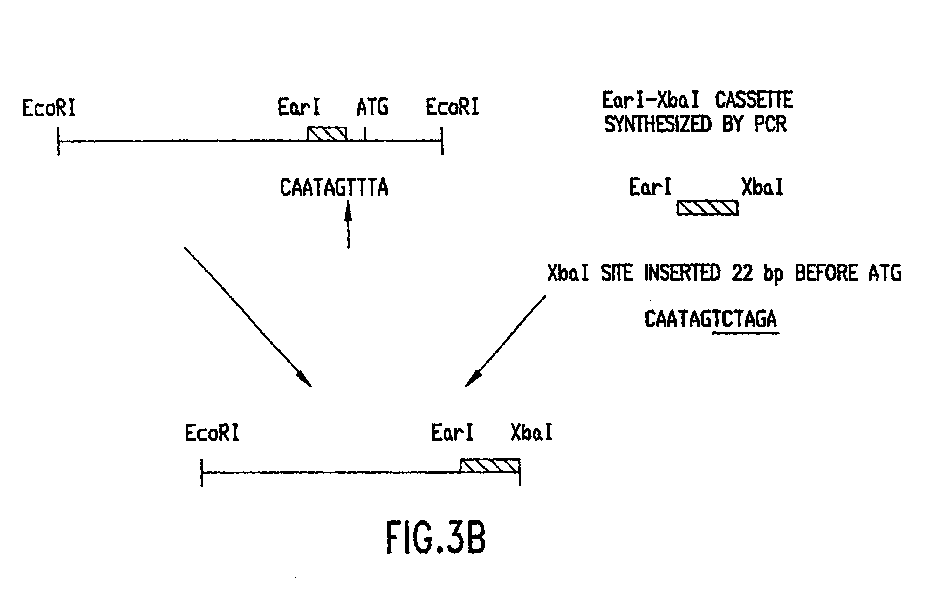

A 210 bp EarI-XbaI fragment (top right of figure 3B) is synthesized by PCR in order to copy the ant43D promoter from 39 bp before the Earl site to 22 bp before the ATG. One of the PCR primers inserts an XbaI site 22 bp before the ATG. A full-length ant43D promoter consisting of the EcoRI-EarI fragment from the original clone and the EarI-XbaI PCR cassette is reassembled in a 3-way ligation into the EcoRI-XbaI sites of bluescript. This promoter clone can be used for transcriptional fusions to coding sequences.

The resulting promoter is excised as a HindIII-XbaI fragment and fused to the GUS gene in PBI101 (pCIB3178). Figure 3B demonstrates how the 1.2 kb flanking region of the ant43D gene is obtained. The 1.2 kb promoter fragment is sufficient to confer anther specific activity.

### Example 19: Fusion of the ant43D promoter sequence to the DTA gene

The 1.2 kb ant32 promoter is excised from pLC251 with HindIII-XbaI and replaced with a HindIII-XbaI ant43D promoter fragment. The resulting plasmid is designated pCIB3179.

The DTA coding sequence is fused in the opposite orientation in pCIB3188. The ant32 promoter is excised with HindIII and XbaI from pLC252 and replaced with the ant43D promoter.

### Example 20: Production of Transgenic Plants

Tobacco leaf discs are transformed with the ant32-GUS (pCIB3132 (2 kb promoter) and pCIB3132B (.6 kb promoter)), ant32-DTA (pLC251 and antisense control (pLC252)), ant43D-GUS (pCIB3178), and ant43D-DTA (pCIB3179 and antisense control(pCIB3188)) constructions and mature transformed plants selected as in Horsch *et al*, (1985). The presence of transforming DNA is confirmed using PCR.

### Example 21: GUS Analysis of ant32 Transgene Expression

Transformants are tested by the GUS histochemical assay as in Koltunow *et al*, (1990) and fluorometrically as in Jefferson, (1987). In the histochemical assay, GUS expression is seen in the tapetal cell layer of the anthers of flower buds 10 to 20 mm long. Expression is also seen in pollen to a lesser extent. Anther, pistil, pollen, leaf and stem tissue are assayed fluorometrically and GUS activity is limited to anther and pollen tissue.

### Example 22: Analysis of ant32-DTA Transgenic Plants

The flower morphology of 13 transgenic plants containing pLC251 and 15 plants of pLC252 is observed. The plants containing pLC251 all had brown, withered anthers and no pollen shed. In contrast, pLC252 transgenic plants had normal anthers and pollen shed. Selfs and backcrosses are done on all plants. In the pLC251 plants, no self pollinations are obtained, but seeds are obtained from backcrosses. Fertility in self and backcross pollinations is normal for pLC252 plants.

Anthers from 14-16mm and 25-30mm long flower buds are fixed, embedded in paraffin, and sections are stained with toluidine blue. The tapetum and pollen sac are destroyed in pLC251 plants, whereas pLC252 plants had normal morphology.

### Example 23: GUS Analysis of ant43D Transgene Expression

Transformants are tested by the GUS histochemical and fluorometric assays. In the histochemical assay, GUS expression is seen in the tapetal cell layer of anthers of buds 14 to 16 mm long, in microspores, and increasingly in the connective and wall tissue of the anther. Anther, pollen, pistil, leaf, sepal, stem and root tissue are assayed fluorometrically. GUS activity is limited to anther and pollen tissue.

### Example 24: Analysis of ant43D-DTA Transgenic plants

The flower morphology of 8 pCIB3179 plants and eight plants of the control pCIB3188 (DTA in antisense orientation) is observed. The pCIB3179 transgenic plants all had nonfunctional anthers as no pollen was shed. Anther size among different plants ranged from normal to shrunken, anther color from green to brown, and anthers from dehiscent to nondehiscent. The control pCIB3188 transgenic plants had normal anther morphology and pollen shed. Selfs and backcrosses are done on the plants. In pCIB3179 plants, pollinations from backcrosses are obtained, but self pollinations are not. Fertilization in pCIB3188 plants is normal.

Anthers from 8-10mm, 10-12mm, 14-16mm and 25-30mm long flower buds are fixed, embedded in paraffin, and sections are stained with toluidine blue. Microspores are absent from pCIB3179 plants as early as in 8-10mm long buds.

### DEPOSIT

The following deposits have been made at the the USDA Agricultural Research Service Culture Collection, Northern Regional Research Center (NRRL) at 1815 North University Street, Peoria, Ill. 61604 in accordance with the requirements of the Budapest Treaty:

| Plasmid | Date of deposit | Date of viability statement | Deposition No |
|---|---|---|---|
| pCIB 3178 | June 16, 1992 | June 22, 1992 | NRRL B-18978 |
| pCIB 3132 | June 16, 1992 | June 22, 1992 | NRRL B-18977 |

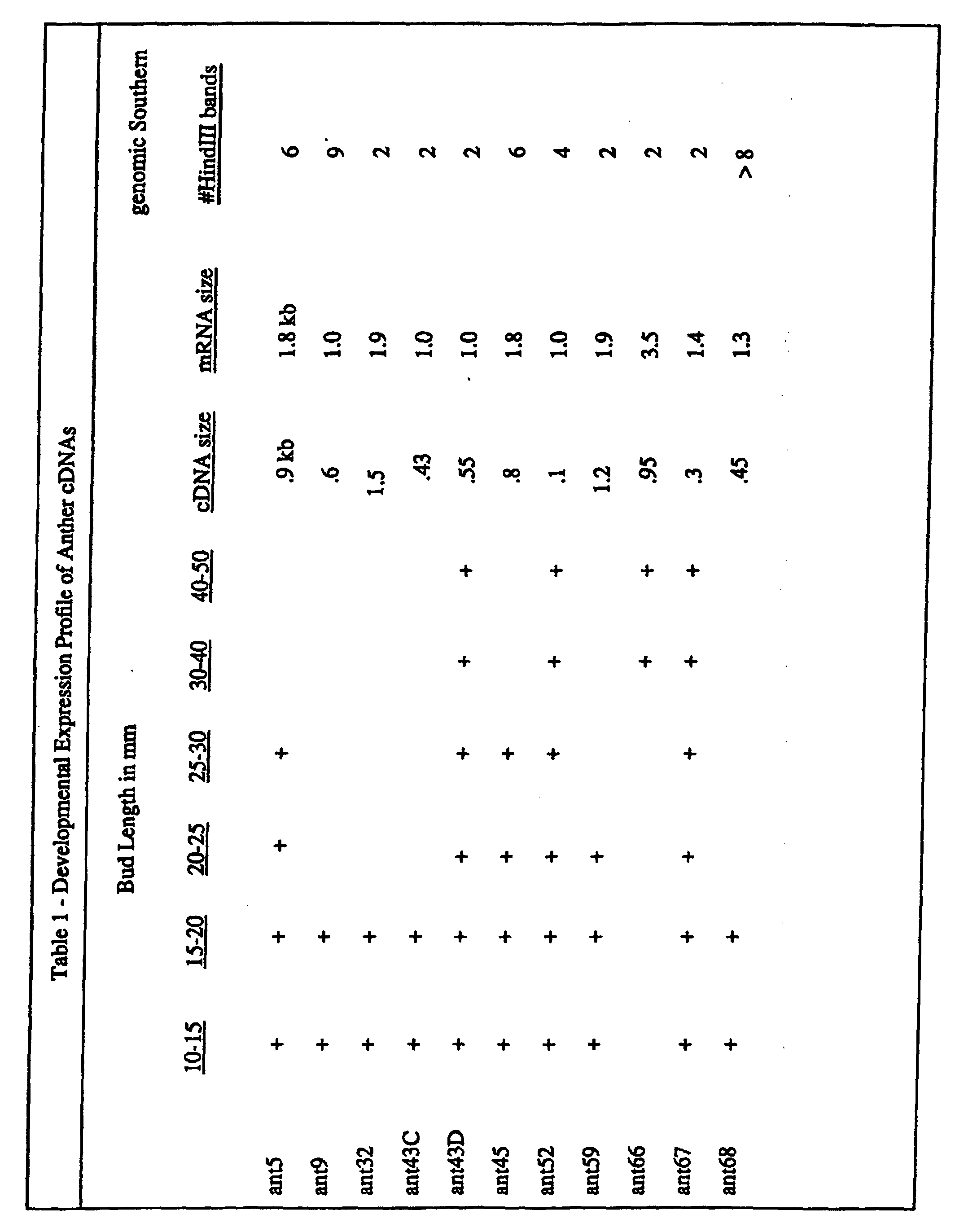

### REFERENCE LISTING

**Benfey** *et al*, EMBO 9: 1677-1684, 1990
**Braun** *et al*, Plant Cell 2: 153, 1990
**Cornellisen** *et al*, EMBO 5: 37-40, 1986
**Della-Cioppa** *et al*, Plant Physiology 84: 965-968, 1987
**Dewey** *et al*, Cell 44: 439, 1986
**Dewey** *et al*, Proc Natl Acad Sci USA, 84: 5374, 1987
**Erlich** (Ed.), PCR Technology, Stockton Press (New York 1989)
**Goodall** G *et al*, Methods in Enzymology 181: 148-161,1990
**Greenfield** *et al*, Proc Natl Acad Sci USA, 80: 6853, 1983
**Grimsley** NH *et al*, Nature 325: 177-179,1987
**Harper** *et al*, Proc Natl Acad Sci, USA 86: 1234-1238
**Heijne** *et al*, Eur J Biochem, 180: 535-545, 1989
**Horsch** *et al*, Science 227: 1229-1231, 1985
**Huang** *et al*, Proc Nat Acad Sci USA, 88: 10716-10720, 1991
**Jefferson,** Plant Mol Biol Rep 5: 387-405, 1987
**Keen** *et al*, J. Bacteriology 168: 595, 1986
**Koltunow** *et al*, Plant Cell 2: 1201-1224,1990
**Maeser** *et al*, Mol Gen Genet 230: 170-176, 1991
**Maniatis** *et al*, Molecular Cloning: A Laboratory Manual, Cold Spring Harbor Laboratory
Press, NY (1982)
**McLean** *et al*, J Bacteriology 169: 1017-1023, 1987
**Mullis** *et al*, Meth Enzymology 155: 335-350, 1987;
**Negrutiu I** *et al*, Plant Mol Biol 8: 363-373, 1987
**Neuhaus** *et al*, Theor Appl Genet 74: 30 -36, 1987
**Palmiter** *et al*, Cell 50: 435-443, 1987
**Perez-Grau** *et al*, Plant Cell 1: 1095-1109, 1989
**Roberto** *et al*, Pro Natl Acad Sci USA, 87: 5795-5801, 1990
**Romano** *et al*, Genes and Development 5: 438-446, 1991
**Sambrook J** *et al*, Molecular Cloning, A Laboratory Manual, Second Ed., Cold Spring Harbor Laboratory Press, 1989
**Sanger** *et al* Proc Natl Acad Sci USA, 74: 5463-5467, 1977
**Schekman**, TIBS, 188, 1985
**Schmitz** *et al*, Plant Cell, 1: 783-791, 1989
**Schocher** RJ *et al*, Bio/Technology 4: 1093-1096, 1986
**Shillito** *et al*, Bio/Technology 3: 1099-1103, 1985
**Spena** *et al*, Mol Gen Genet 227: 205-212, 1991
**Wang Y-C** *et al*, Plant Mol Biol 11: 433-439, 1988

### Patent Literature

EP-A 0420 819 A1
US-P 4,918,006
US-P 5,086,169
PCT WO 89/10396
PCT WO 90/08825
PCT WO 90/08831
PCT WO 90/08828

## Claims

1. An isolated nucleotide sequence consisting of an anther-specific genomic DNA sequence consisting of SEQ. ID No. 18.

2. An isolated genomic DNA sequence which is capable of hybridizing with the anther-specific DNA sequence of claim 1 under stringent conditions and which functions as a promoter of anther-specific transcription of associated expressible and preferably coding DNA sequences in recombinant or chimeric DNA constructs.

3. An isolated DNA sequence according to claim 2, wherein said sequence comprises base 1 to base 1229 or base 1 to 1147 of SEQ ID No: 18.

4. A recombinant DNA sequence comprising, in a 5' to 3' direction, a promoter region according to claim 2 or 3 operatively linked to an expressible DNA sequence.

5. A recombinant DNA sequence comprising, in a 5' to 3' direction, a promoter region according to claim 2 or 3, operatively linked to a signal sequence, which is operatively linked to an expressible DNA sequence.

6. A recombinant DNA sequence according to any one of claims 4 or 5, wherein the expressible DNA sequence is a coding sequence.

7. A recombinant DNA sequence according to claim 6, wherein the coding DNA sequence encodes a polypeptide which will disrupt formation of viable pollen when expressed in the anther cells.

8. A recombinant DNA sequence according to claim 7, wherein the coding DNA sequence encodes a polypeptide selected from the group consisting of DTA, TURF-13, pectate lyase, gin recombinase, iaaL and cytA toxin.

9. A plasmid comprising a recombinant DNA molecule according to any one of claims 4 to 8.

10. The plasmid of claim 9 which is plasmid pCIB3178 deposited under deposit no. NRRL B-18978

11. A promoter fragment capable of hybridizing with the plasmid of claim 10 under stringent conditions and which functions as a promoter of anther-specific transcription of associated expressible and preferably coding DNA sequences in recombinant or chimeric DNA constructs.

12. A recombinant DNA sequence comprising, in a 5' to 3' direction, the promoter fragment of claim 11 operably linked to an expressible DNA sequence.

13. A recombinant DNA sequence comprising, in a 5' to 3' direction, the promoter fragment of claim 11, operably linked to a signal sequence, which is operably linked to an expressible DNA sequence.

14. A recombinant DNA sequence according to any one of claims 12 or 13, wherein the expressible DNA sequence is a coding sequence.

15. A recombinant DNA sequence according to claim 14, wherein the coding DNA sequence encodes a polypeptide which will disrupt formation of viable pollen when expressed in the anther cells.

16. A recombinant DNA sequence according to claim 15, wherein the coding DNA sequence encodes a polypeptide selected from the group consisting of DTA, TURF-13, pectate lyase, gin recombinase, iaaL and cytA toxin.

17. A host cell comprising a recombinant DNA molecule according to any one of claims 4 to 8 or 12 to 16.

18. A host cell according to claim 17 **characterized in that** it is a bacterium.

19. A host cell according to claim 17 **characterized in that** it is a plant cell.

20. A transgenic plant and the sexual and/or asexual progeny thereof, which has been transformed with the recombinant DNA sequence according to any one of claims 4 to 6.

21. A transgenic plant and the sexual and/or asexual progeny thereof, which has been transformed with the recombinant DNA sequence according to any one of claims 12 to 14.

22. A transgenic, male-sterile plant which has been transformed with the recombinant DNA sequence according to any one of claims 7 or 8.

23. A transgenic, male-sterile plant which has been transformed with the recombinant DNA sequence according to any one of claims 15 or 16.

24. A process for the production of a recombinant DNA molecule comprising, in a 5' to 3' direction, an anther-specific promoter region according to claim 2 or 3 operatively linked to an expressible DNA sequence, which process comprises
(a) first isolating from a suitable source or synthesising by means of known processes a promoter region responsible for the anther-specific expression of an associated expressible DNA sequence; and
(b) operably linking the said anther-specific DNA sequence in a 5' to 3' direction to an expressible DNA sequence.

25. A process for the production of transformed plant material, including whole plants, comprising a recombinant DNA molecule comprising, in a 5' to 3' direction, an anther-specific promoter region according to claim 2 or 3 operatively linked to an expressible DNA sequence, which process essentially comprises:
(a) first isolating from a suitable source or synthesising by means of known processes a promoter region responsible for the anther-specific expression of an associated expressible DNA sequence;
(b) operably linking the said anther-specific DNA sequence in a 5' to 3' direction to an expressible DNA sequence;
(c) cloning the final construct into a plant expression vector under the control of expression signals active in plants;
(d) transforming the said expression vector into plant material by means of known processes and expressing it therein; and optionally
(e) regenerating the plant material transformed according to step (d) to a whole and preferably phaenotypically normal plant.

26. An isolated nucleotide sequence consisting of the anther-specific cDNA sequence shown in SEQ. ID No. 3, wherein said cDNA sequence is **obtained** from the anther-specific genomic sequence of claim 1.

## Patentansprüche

1. Isolierte Nucleotidsequenz aus einer Anthere-spezifischen Genom-DNA-Sequenz bestehend aus SEQ. ID Nr. 18.

2. Isolierte Genom-DNA-Sequenz mit der Fähigkeit zur Hybridisierung mit der Anthere-spezifischen DNA-Sequenz nach Anspruch 1 unter stringenten Bedingungen, die als Promotor einer Anthere-spezifischen Transkription assoziierter exprimierbarer und vorzugsweise codierender DNA-Sequenzen in rekombinanten oder chimären DNA-Konstrukten fungiert.

3. Isolierte DNA-Sequenz nach Anspruch 2, wobei die Sequenz Base 1 bis Base 1229 oder Base 1 bis 1147 von SEQ. ID Nr. 18 umfasst.

4. Rekombinante DNA-Sequenz, die in einer 5'- zu 3'-Richtung eine Promotorregion nach Anspruch 2 oder 3 in operativer Verknüpfung mit einer exprimierbaren DNA-Sequenz umfasst.

5. Rekombinante DNA-Sequenz, die in einer 5'- zu 3'-Richtung eine Promotorregion nach Anspruch 2 oder 3 in operativer Verknüpfung mit einer Signalsequenz umfasst, die operativ mit einer exprimierbaren DNA-Sequenz verknüpft ist.

6. Rekombinante DNA-Sequenz nach einem der Ansprüche 4 oder 5, wobei die exprimierbare DNA-Sequenz eine codierende Sequenz ist.

7. Rekombinante DNA-Sequenz nach Anspruch 6, wobei die codierende DNA-Sequenz für ein Polypeptid codiert, das die Bildung lebensfähiger Pollen bei Expression in den Antherezellen unterbricht.

8. Rekombinante DNA-Sequenz nach Anspruch 7, wobei die codierende DNA-Sequenz für ein Polypeptid codiert, das aus der Gruppe ausgewählt ist, die aus DTA. TURF-13, Pectatlyase, Ginrekombinase, iiaL und cytA-Toxin besteht.

9. Plasmid, das ein rekombinantes DNA-Molekül nach einem der Ansprüche 4 bis 8 umfasst.

10. Plasmid nach Anspruch 9, das das Plasmid pCIB3178 ist, das unter der Hinterlegungsnummer NRRL B-18978 hinterlegt ist.

11. Promotorfragment mit der Fähigkeit zur Hybridisierung mit dem Plasmid nach Anspruch 10 unter stringenten Bedingungen, das als Promotor einer Anthere-spezifischen Transkription von assoziierten exprimierbaren und vorzugsweise codierenden DNA-Sequenzen in rekombinanten oder chimären DNA-Konstrukten fungiert.

12. Rekombinante DNA-Sequenz, die in einer 5'- zu 3'-Richtung das Promotorfragment nach Anspruch 11 in operativer Verknüpfung mit einer exprimierbaren DNA-Sequenz umfasst.

13. Rekombinante DNA-Sequenz, die in einer 5'- zu 3'-Richtung das Promotorfragment nach Anspruch 11 in operativer Verknüpfung mit einer Signalsequenz umfasst, die operativ mit einer exprimierbaren DNA-Sequenz verknüpft ist.

14. Rekombinante DNA-Sequenz nach einem der Ansprüche 12 oder 13, wobei die exprimierbare DNA-Sequenz eine codierende Sequenz ist.

15. Rekombinante DNA-Sequenz nach Anspruch 14, wobei die codierende DNA-Sequenz für ein Polypeptid codiert, das die Bildung von lebensfähigen Pollen bei Expression in den Antherezellen unterbricht.

16. Rekombinante DNA-Sequenz nach Anspruch 15, wobei die codierende DNA-Sequenz für ein Polypeptid codiert, das aus der Gruppe ausgewählt ist, die aus DTA, TURF-13, Pectatlyase, Ginrekombinase, iiaL und cytA-Toxin besteht.

17. Wirtzelle, die ein rekombinantes DNA-Molekül nach einem der Ansprüche 4 bis 8 oder 12 bis 16 umfasst.

18. Wirtzelle nach Anspruch 17, die **dadurch gekennzeichnet ist, dass** sie ein Bakterium ist.

19. Wirtzelle nach Anspruch 17, die **dadurch gekennzeichnet ist, dass** sie eine Pflanzenzelle ist.

20. Transgene Pflanze und die sexuellen und/oder asexuellen Nachkommen hiervon, die mit der rekombinanten DNA-Sequenz nach einem der Ansprüche 4 bis 6 transformiert wurde.

21. Transgene Pflanze und die sexuellen und/oder asexuellen Nachkommen hiervon, die mit der rekombinanten DNA-Sequenz nach einem der Ansprüche 12 bis 14 transformiert wurde.

22. Transgene männlich sterile Pflanze, die mit der rekombinanten DNA-Sequenz nach einem der Ansprüche 7 oder 8 transformiert wurde.

23. Transgene männlich sterile Pflanze, die mit der rekombinanten DNA-Sequenz nach einem der Ansprüche 15 oder 16 transformiert wurde.

24. Verfahren zur Herstellung eines rekombinaten DNA-Moleküls, das in einer 5'- zu 3'-Richtung eine Anthere-spezifische Promotorregion nach Anspruch 2 oder 3 in operativer Verknüpfung mit einer exprimierbaren DNA-Sequenz umfasst, wobei das Verfahren die folgenden Stufen umfasst:
(a) anfängliches Isolieren aus einer geeigneten Quelle oder Synthetisieren mit Hilfe bekannter Verfahren einer Promotorregion, die für die Anthere-spezifische Expression einer assoziierten exprimierbaren DNA-Sequenz verantwortlich ist, und
(b) operatives Verknüpfen der Anthere-spezifischen DNA-Sequenz in einer 5'- zu 3'-Richtung mit einer exprimierbaren DNA-Sequenz.

25. Verfahren zur Herstellung von transformierten Pflanzenmaterial einschließlich ganzer Pflanzen, das bzw. die ein rekombinantes DNA-Molekül umfasst bzw. umfassen, das in einer 5'- zu 3'-Richtung eine Anthere-spezifische Promotorregion nach Anspruch 2 oder 3 in operativer Verknüpfung mit einer exprimierbaren DNA-Sequenz umfasst, wobei das Verfahren im Wesentlichen die folgenden Stufen umfasst:
(a) anfängliches Isolieren aus einer geeigneten Quelle oder Synthetisieren nach bekannten Verfahren einer Promotorregion, die für die Anthere-spezifische Expression einer assoziierten exprimierbaren DNA-Sequenz verantwortlich ist,
(b) operatives Verknüpfen der Anthere-spezifischen DNA-Sequenz in einer 5'- zu 3'-Richtung mit einer exprimierbaren DNA-Sequenz,
(c) Klonieren des Endkonstrukts in einen Pflanzenexpressionsvektor unter der Steuerung von Expressionssignalen, die in Pflanzen aktiv sind,
(d) Transformieren des Expressionsvektors in Pflanzenmaterial mit Hilfe bekannter Verfahren und Exprimieren desselben darin und optional
(e) Regenerieren des gemäß Stufe (d) transformierten Pflanzenmaterials zu einer ganzen und vorzugsweise phaenotypisch normalen Pflanze.

26. Isolierte Nucleotidsequenz aus der Anthere-spezifischen cDNA-Sequenz, die in SEQ. ID Nr. 3 angegeben ist, wobei die cDNA-Sequenz aus der Anthere-spezifischen Genomsequenz nach Anspruch 1 erhalten wurde.

## Revendications

1. Séquence nucléotidique isolée constituée d'une séquence d'ADN génomique spécifique à l'anthère constituée de SEQ ID n° : 18.

2. Séquence d'ADN génomique isolée qui est capable de s'hybrider avec une séquence d'ADN spécifique à l'anthère selon la revendication 1 dans des conditions stringentes, et qui agit en tant que promoteur de la transcription spécifique à l'anthère de séquences d'ADN associées exprimables et de préférence codantes, dans des constructions d'ADN chimérique ou recombinant.

3. Séquence d'ADN isolée selon la revendication 2, dans laquelle ladite séquence comprend la base 1 à la base 1229 ou le base 1 à la base 1147 de la SEQ ID n° : 18.

4. Séquence d'ADN recombinant comprenant, dans une direction de 5' en 3', une région promotrice selon la revendication 2 ou la revendication 3, liée de façon opérationnelle à une séquence d'ADN exprimable.

5. Séquence d'ADN recombinant comprenant, dans une direction de 5' en 3', une région promotrice selon la revendication 2 ou la revendication 3, liée de façon opérationnelle à une séquence signal, qui est liée de façon opérationnelle à une séquence d'ADN exprimable.

6. Séquence d'ADN recombinant selon l'une quelconque des revendications 4 ou 5, dans laquelle la séquence d'ADN exprimable est une séquence codante.

7. Séquence d'ADN recombinant selon la revendication 6, dans laquelle la séquence d'ADN codant code pour un polypeptide qui détruira la formation de pollen viable quand il est exprimé dans des cellules d'anthère.

8. Séquence d'ADN recombinant selon la revendication 7, dans laquelle la séquence d'ADN codant code pour un polypeptide choisi dans le groupe constitué par DTA, TURF-13, la pectate lyase, la gin recombinase, iaaL et la toxine cytA.

9. Plasmide comprenant une molécule d'ADN recombinant selon l'une quelconque des revendications 4 à 8.

10. Plasmide selon la revendication 9, qui est le plasmide pCIB3178 déposé sous le n° de dépôt NRRL B-18978.

11. Fragment promoteur capable de s'hybrider avec le plasmide selon la revendication 10, dans des conditions astringentes, et qui agit en tant que promoteur de la transcription spécifique à l'anthère de séquences d'ADN associées exprimables, et de préférence codantes, dans des constructions d'ADN recombinant ou chimérique.

12. Séquence d'ADN recombinant comprenant, dans une direction de 5' en 3', le fragment promoteur selon la revendication 11, lié de façon opérationnelle à une séquence d'ADN exprimable.

13. Séquence d'ADN recombinant comprenant, dans une direction de 5' en 3', le fragment promoteur selon la revendication 11, lié de façon opérationnelle à une séquence signal, qui est liée de façon opérationnelle à une séquence d'ADN exprimable.

14. Séquence d'ADN recombinant selon l'une quelconque des revendications 12 ou 13, dans laquelle la séquence d'ADN exprimable est une séquence codante.

15. Séquence d'ADN recombinant selon la revendication 14, dans laquelle la séquence d'ADN codant code pour un polypeptide qui détruira la formation de pollen viable quand il est exprimé dans des cellules d'anthère.

16. Séquence d'ADN recombinant selon la revendication 15, dans laquelle la séquence d'ADN codant code pour un polypeptide choisi dans le groupe constitué par DTA, TURF-13, la pectate lyase, la gin recombinase, iaaL et la toxine cytA.

17. Cellule hôte comprenant une molécule d'ADN recombinant selon l'une quelconque des revendications 4 à 8 ou 12 à 16.

18. Cellule hôte selon la revendication 17, **caractérisée en ce qu'**elle est une bactérie.

19. Cellule hôte selon la revendication 17, **caractérisée en ce qu'**elle est une cellule de plante.

20. Plante transgénique et sa progéniture sexuée et/ou asexuée, qui a été transformée avec la séquence d'ADN recombinant selon l'une quelconque des revendications 4 à 6.

21. Plante transgénique et sa progéniture sexuée et/ou asexuée, qui a été transformée avec la séquence d'ADN recombinant selon l'une quelconque des revendications 12 à 14.

22. Plante transgénique, mâle stérile qui a été transformée avec la séquence d'ADN recombinant selon l'une quelconque des revendications 7 ou 8.

23. Plante transgénique, mâle stérile qui a été transformée avec la séquence d'ADN recombinant selon l'une quelconque des revendications 15 ou 16.

24. Procédé pour la production d'une molécule d'ADN recombinant comprenant, dans une direction de 5' en 3', une région promotrice spécifique à l'anthère selon la revendication 2 ou la revendication 3, liée de façon opérationnelle à une séquence d'ADN exprimable, lequel procédé comprend :
(a) d'abord isoler à partir d'une source convenable ou synthétiser au moyen de procédés connus, une région promotrice responsable de l'expression spécifique à l'anthère d'une séquence d'ADN associée exprimable ; et
(b)lier de façon opérationnelle ladite séquence d'ADN spécifique à l'anthère selon une direction de 5' en 3' , à une séquence d'ADN exprimable.

25. Procédé pour la production d'un matériau de plante transformé, y compris des plantes complètes, comprenant une molécule d'ADN recombinant comprenant, dans une direction de 5' en 3', une région promotrice spécifique à l'anthère selon la revendication 2 ou la revendication 3, liée de façon opérationnelle à une séquence d'ADN exprimable, lequel procédé comprend :
(a) d'abord isoler à partir d'une source convenable ou synthétiser au moyen de procédés connus, une région promotrice responsable de l'expression spécifique à l'anthère d'une séquence d'ADN associée exprimable ;
(b) lier de façon opérationnelle ladite séquence d'ADN spécifique à l'anthère dans une direction de 5' en 3' à une séquence d'ADN exprimable ;
(c) cloner la construction finale dans un vecteur d'expression de plante, sous le contrôle de signaux d'expression actifs chez les plantes ;
(d) transformer ledit vecteur d'expression dans le matériau de plante au moyen de procédés connus, et l'exprimer dedans ; et facultativement
(e) régénérer le matériau de plante transformé selon l'étape (d) en une plante complète et, de préférence, phénotypiquement normale.

26. Séquence nucléotidique isolée constituée de la séquence d'ADNc spécifique à l'anthère représentée dans SEQ ID n° : 3, dans laquelle ladite séquence d'ADNc est obtenue à partir d'une séquence génomique spécifique à l'anthère selon la revendication 1.
